# FASCICULE DE BREVET EUROPEEN

(11) **EP 1 216 074 B1**
(45) Date de publication et mention de la délivrance du brevet: **10.01.2007**
(21) Numéro de dépôt: 00958752.8
(22) Date de dépôt: 28.08.2000
(51) Int. Cl.: A61N 1/32

(54) **APPAREIL DE STIMULATION ELECTRIQUE DU SYSTEME LYMPHATIQUE**
GERÄT ZUR ELEKTRISCHEN STIMULATION DES LYMPHSYSTEMS
APPARATUS FOR ELECTRICAL STIMULATION OF THE LYMPHATIC SYSTEM

(30) Priorité: 01.09.1999 FR 9911043
(43) Date de publication de la demande: 26.06.2002
(73) Titulaire: Physiomed Elektromedizin AG, 91220 Schnaittach/Laipersdorf (DE)
(72) Inventeur: PUJOL, Laurent, F-95528 Cergy Pontoise Cedex (FR)
(74) Mandataire: Schneck, Herbert
(86) Numéro de dépôt international: PCT/FR2000/002388
(87) Numéro de publication internationale: WO 2001/015772

(56) Documents cités:
- EP-A- 0 788 811
- DE-A- 3 716 816
- FR-A- 2 617 722
- US-A- 5 725 563
- US-A- 5 817 138
- US-A- 5 935 156

## Description

La présente invention concerne le secteur technique de la stimulation électrique de certains tissus.

Ces techniques consistent à envoyer vers les tissus sélectionnés des excitations électriques, généralement répétitives sous forme de « trains » d'excitation, adaptés afin de provoquer localement une excitation du tissu considéré, qui se traduit par un effet bénéfique.

L'invention s'applique plus précisément à la stimulation du système lymphatique, en vue d'améliorer, stimuler ou restaurer le drainage lymphatique car tout l'équilibre des fluides du corps en dépend, dont le système circulatoire et notamment veineux.

### Généralités :

Le système lymphatique ou lymphatico-veineux est chargé de transporter les liquides et les toxines et les déchets en excès dans l'organisme, de les filtrer et de les évacuer dans la circulation générale. C'est le système d'épuration du corps ; lorsque la lymphe circule correctement, les canaux ne sont pas engorgés. Au contraire, lorsque pour diverses raisons, surmenage, fatigue, obésité, âge (perte d'élasticité des tissus), pathologie, exposition à la pollution, facteurs génétiques, facteurs aggravants comme le tabac, etc...., la circulation lymphatique ralentit, les tissus interstitiels commencent à se gorger de toxines et d'eau. On observe alors des phénomènes bien connus tels que
- Vieillissement des tissus :
   poches sous les yeux, rides, flaccidité de la peau en général
- Pathologie :
   Lymphostase par
      - aplasie ou agénésie
      - causes iatrogènes
      - débordement du système lymphatique, en cas de surfonction due à un problème veineux majeur
- Cellulite :
   stade 1 = stade veino lymphatique ; la lymphe se place dans la couche de graisse qui est hydrophobe - stade mobile ;
   stade 2 = infiltrat ; la lymphe se place dans les cloisons autour des cellules - stade d'installation ;
   stade 3 = fibrose tendant à la sclérose ; gélification du stade 2 avec, progressivement, sclérose du tissu, tensions qui donnent l'aspect « peau d'orange » - stade peu mobile, voire sclérose.

Il est donc important de pouvoir stimuler ce système, de manière spécifique et contrôlée, afin d'obtenir ou d'améliorer notamment le « drainage lymphatique », c'est à dire de stimuler selon un trajet connu du praticien les canaux lymphatiques au moyens de cycles de stimulation et de relaxation. Ces opérations mobilisent la chaîne ganglionnaire par appel de la lymphe et « foulage » pour la résorber.

Au plan des tissus musculaires, on distingue globalement les muscles blancs ou lisses des muscles rouges striés squelettiques.

On sait que les muscles blancs ou muscles organiques sont ceux des organes, notamment de l'intestin grêle, de l'estomac, et autres organes connus.

On sait également que les veines, elles, comportent très peu de muscles blancs.

On sait également que les muscles lisses ou blancs, qui sont peu ou pas présents à l'intérieur des veines (partie médiane ou tunique médiane ou médiaveineuse), ne comportent pas de stries.

La veine n'a donc pas de mobilité propre. Ce sont les muscles rouges extérieurs à la veine qui vont permettre d'exercer une action mécanique sur la veine (appelée vis a latere / effet dit veino-pompe).

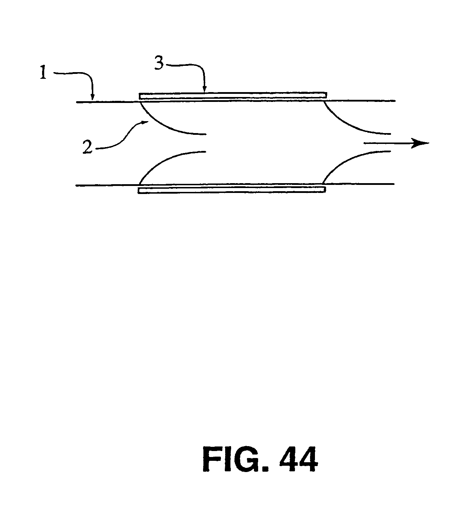

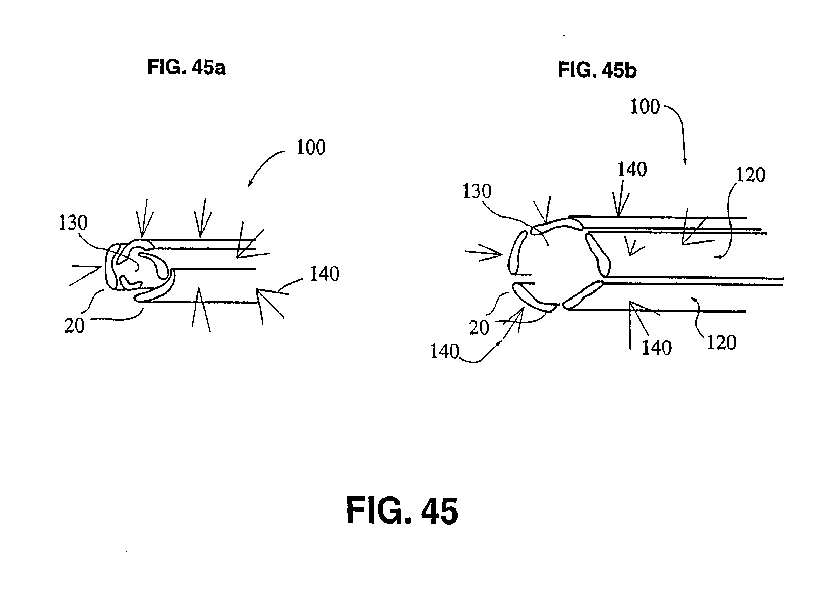

Par contre, les collecteurs lymphatiques comportent des muscles blancs (cf. Fig.44: le « lymphagion » et Fig. 45). C'est une des grandes différences avec le système veineux (pas de varices lymphatiques) et cela rend donc ce système potentiellement dynamisable mécaniquement et électriquement.
- les collecteurs primaires, par leur système de haubans reliés au tissu, cf. fig. 45, seront eux dynamisés par étirement ou dépression de la peau ;
- les ganglions lymphatiques ne sont pas musclés; leur stimulation est donc beaucoup plus difficile.

On a représenté sur la figure 45 annexée, qui se compose des figures 45 A et 45 B, schématiquement, les « précollecteurs » en phase de repos (figure 45 A) et en phase de travail (figure 45 B). On voit que les parois 120 des précollecteurs sont connectées au tissu conjonctif ou « interstitium » (dont le détail n'est pas représenté) par des « haubans » 140.

Ces haubans étant fixés dans les tissus, toute dépression, toute augmentation de volume, tout étirement, va étirer ces haubans et donc augmenter la capacité de drainage.

Un étirement des tissus peut être réalisé par :
- mouvement d'un membre,
- augmentation de volume d'un membre lors d'un oedème,
- adaptation à l'effort (mouvement + augmentation de la perfusion et du liquide circulant.

En phase de repos, les parois 120 sont peu jointives et la « lumière » 130 du précollecteur est réduite. En phase de travail, au contraire, les parois restent peu jointives mais l'étirement des haubans 140 provoque une augmentation de la « lumière » 130.

Le problème technique de la stimulation du système lymphatique est donc radicalement différent de celui concernant le système veineux.

Le système d'évacuation des déchets, y compris le système lymphatique se compose et fonctionne schématiquement de la manière suivante.

Partant d'un système artériel qui va amener dans les tissus des nutriments, de l'oxygène, et des liquides, il existera un système d'évacuation des déchets, de renouvellement des liquides.
- Le système d'évacuation sera double :
   - le système veineux = système rapide de récupération des liquides, des petits déchets (CO₂, etc.) avec maintien de là pression osmotique et onchotique dans les tissus selon la loi de Starling 1 er et 2^{ème}.
   - le système lymphatique : système beaucoup plus lent de gestion des liquides et de l'élimination des gros déchets, et participe à l'immunologie en transportant les lymphocytes qui font partie du système immunitaire.
- La lymphe c'est le liquide d'imbibition des tissus. 80 % de cette lymphe se trouve entre l'épiderme et l'aponévrose musculaire superficielle.
- Le réseau lymphatique représente 500 m2 de superficie d'échange et d'évacuation (système découpé à plat).

Le système lymphatique est le véritable gestionnaire de l'hydratation ainsi que « l'éboueur » de ces tissus, et participe de plus à l'immunologie comme expliqué ci-dessus..

Ce système est composé dans ses tissus d'une majorité de muscles, contrairement au système veineux.

Partant de fentes vasculaires, la résille lymphatique est composée en arborisation terminale de collecteurs primaires à abouchements directs ou indirects, constitués de cellules peu jointives, reliées aux tissus par des haubans ; toute argumentation de volume, tout déplacement des tissus, vont étirer ces haubans, ouvrir la lumière de ces tubules et, par l'augmentation de pression, la lymphe va se trouver entraînée dans le système.
- L'étape suivante va permettre à la lymphe de circuler dans des précollecteurs, le tubule lymphatique commence à s'endothélialiser et enfin la lymphe arrive au niveau des collecteurs lymphatiques.
   Le collecteur lymphatique est un vaisseau organisé particulier tel que représenté sur la Figure 44. Ce collecteur est muni de valvules qui donnent un sens obligatoire au courant lymphatique. Entre ces valvules, le tissu tubulaire constitué de muscles blancs (soumis au système neurovégétatif sympathique et parasympathique) est parcouru, dans le sens disto-proximal, par une onde péristaltique - contraction musculaire de chaque lymphangion sur un rythme lent automatique (possibilité de variations sympathiques et parasympathiques).
- Ces collecteurs vont arriver aux ganglions (vaisseaux afférents).
- Ces ganglions ne sont pas musclés ; il s'agit d'une véritable « usine de retraitement des déchets », et ils vont agir par imbibition de la lymphe dans le tissu ganglionnaire.
   Deux systèmes de nettoyage de la lymphe :
   - système phagocytaire = des phagocytes vont « digérer » les éléments inertes (sans matériel génétique) en molécules simples,
   - système immunitaire = reconnaissance d'un matériel génétique différent (bactéries, virus, métastases...) les macrophages et lymphocytes vont « tuer » ces cellules différentes et désorganiser l'ADN pour libérer les liaisons aminées et redonner dans la circulation des acides simples non combinés, inoffensifs, et même réutilisables par le corps pour fabriquer de nouvelles chaînes d'ADN à son image.
- Un système d'adaptation à l'effort est constitué d'une double circulation, lente et rapide :
   - lente = voir ci-dessus
   - rapide = une partie de la lymphe n'est pas traitée sur un premier ganglion mais sur les suivants (celle traitée en voie lente au début passera sur voie rapide ensuite). A l'arrivée au coeur, la lymphe est « propre », les vaisseaux qui repartent des ganglions sont nommés : vaisseaux afférents.

Le système lymphatique ou lymphatico-veineux est chargé de transporter les liquides et les toxines en excès dans l'organisme, de les filtrer et de les évacuer dans la circulation lymphatique et sanguine. C'est le système dépuration du corps; lorsque la lymphe circule correctement, les canaux ne sont pas engorgés. Au contraire, lorsque pour diverses raisons, surmenage, fatigue, obésité, âge (perte d'élasticité des tissus), pathologie, exposition à la pollution, facteurs génétiques, facteurs aggravants comme le tabac, etc...., la circulation lymphatique ralentit, les tissus interstitiels commencent à se gorger de toxines. On observe alors des phénomènes bien connus tels que formation de poches sous les yeux, de rides du visage, etc.... ainsi que phlébites, gonflement des membres, etc....

Ce système lymphatique est donc bien le gestionnaire de la qualité des tissus, et de la bonne défense immunologique du corps, objet des buts thérapeutiques visés par l'invention.

### On connaît dans ce domaine les brevets suivants:

FR 2 541 119 (Klotz) destiné à stimuler les muscles lisses par des impulsions progressives, comme le USP 4 177 819 ou le FR 2 433 950, ou le FR 2 528 709, ou le USP 4 068 669 ou le USP 3 645 267, ou le USP 3 050 695, ou le USP 3 077 884 ou enfin le USP 4 167 189 qui mesure l'impédance et applique un signal en forme de vague et le EP 0 057 561, EP 0 148 312, EP 0 425 673 et FR 2 704 151 visant à effectuer ' une mesure d'impédance.

L'art antérieur le plus proche consiste, de l'avis du Demandeur, en les brevets suivants :

FR 2 541,119, FR 2 704 151, USP 4 167 189, EP 0 425 673. Ces technologies sont présentées comme stimulant les muscles blancs - lisses - de la média veineuse et travaillent toujours sur le système veineux.

On connaît également le brevet WO 91 07207 qui décrit l'application d'un courant pulsé pour le drainage lymphatique.

Le document FR 2 617 722 A décrit un appareil de stimulation du système lymphatique. Cet appareil comprend un générateur d'impulsions électriques et une série d'électrodes que l'on place sur l'épiderme du sujet à traiter, de manière à faire passer dans le corps considéré des trains de stimulations électriques. Chaque stimulation ou impulsion n'est pas de signal carré. Le temps d'excitation est plus court que le temps de repos ou relaxation.

Il a maintenant été découvert que l'on pouvait stimuler le système lymphatique ou lymphatico-veineux, chez l'homme ou l'animal, notamment le cheval, au moyen de stimulations électriques spécifiques qui seront décrites ci-après.

L'invention concerne un nouvel appareil de stimulation du système lymphatique chez l'être humain ou les animaux, notamment le cheval, comme défini dans la revendication 1.

Selon un mode de mise en oeuvre particulier, l'intensité du courant électrique appliqué sera inférieure ou égale à 1 mA (milli Ampère), et sera de préférence de l'ordre de 6 à 300 µA (micro Ampère) ou plus en fonction du nombre d'électrodes qui, avec le « ressenti » par le patient, constituent les seules limites en la matière.

Selon un mode préféré de mise en oeuvre, on appliquera des trains d'impulsions ou « salves » de 5 / 5 à 10 / 10 ou à 15 / 15, de préférence à 10 / 10.

Chaque train d'impulsions présente une inversion de polarité par rapport au précédent. La moyenne du courant est donc nulle ce qui évite toute polarisation de matériel dans le tissu traité.

Selon un mode de mise en oeuvre particulier, la fréquence de chaque impulsion sera comprise entre 0,1 et 3 Hz, et sera de préférence comprise entre 0,7 et 2,5 Hz, de préférence voisine de 1,5 ou 2 Hz.

D'autres caractéristiques et avantages de l'invention apparaîtront mieux à la lecture de la description qui va suivre, et en se référant aux essais et au dessin annexé, sur lequel:

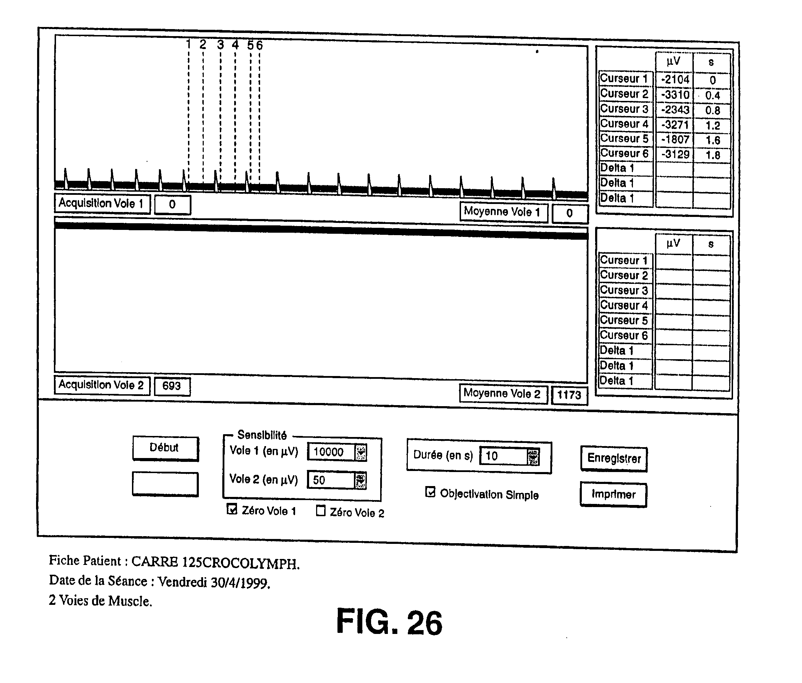

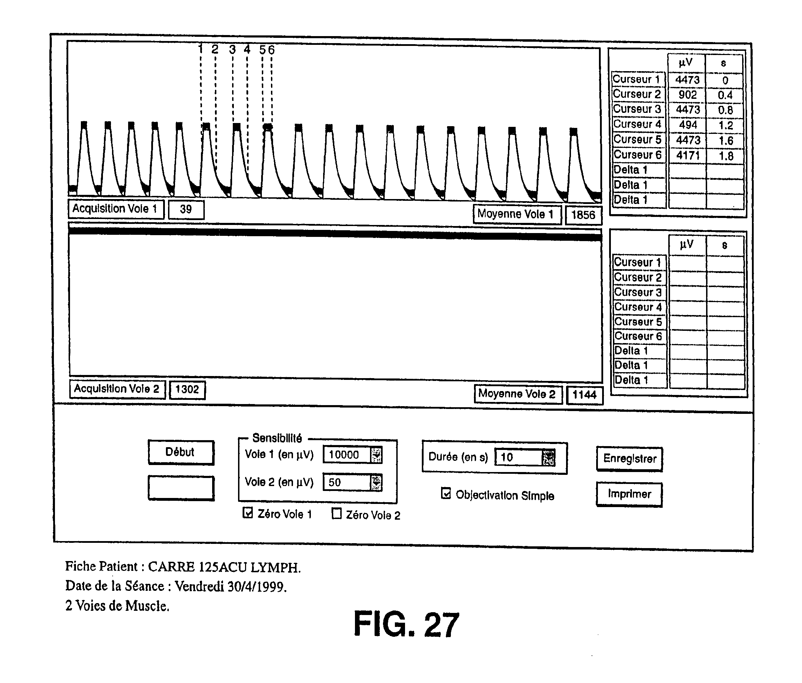

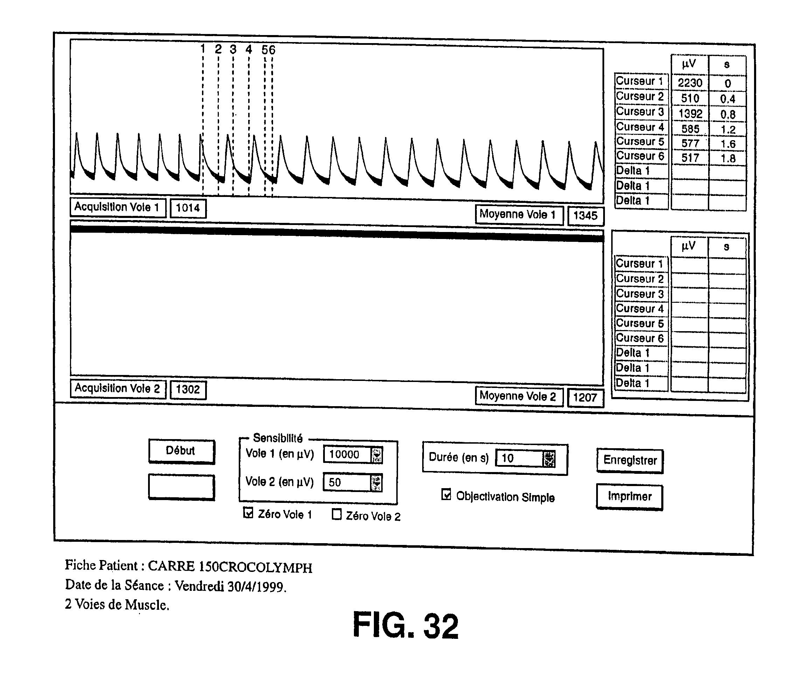

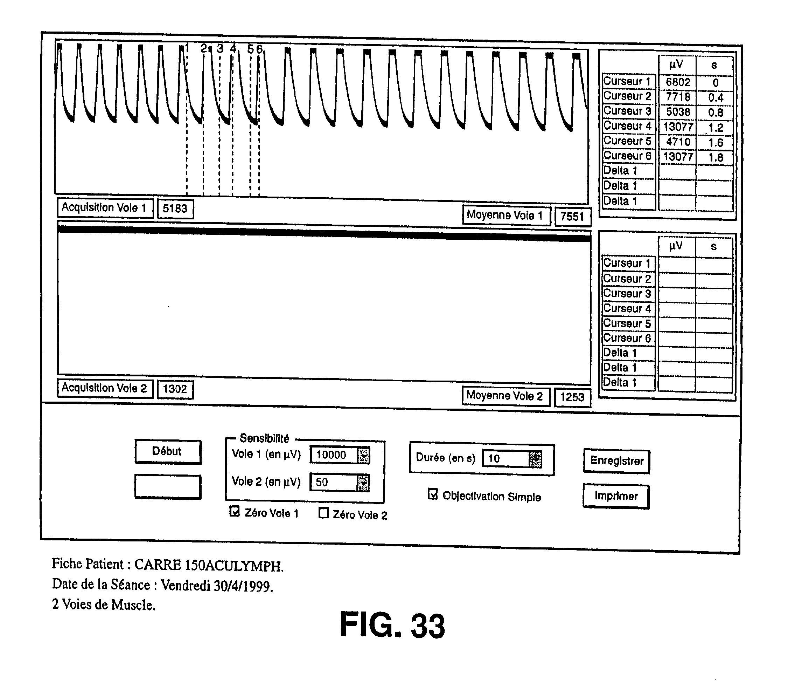

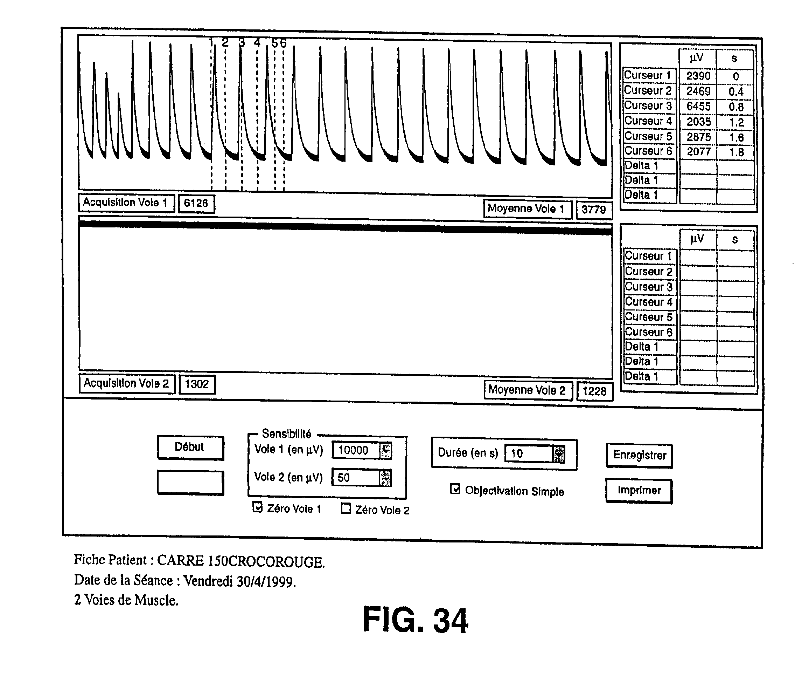

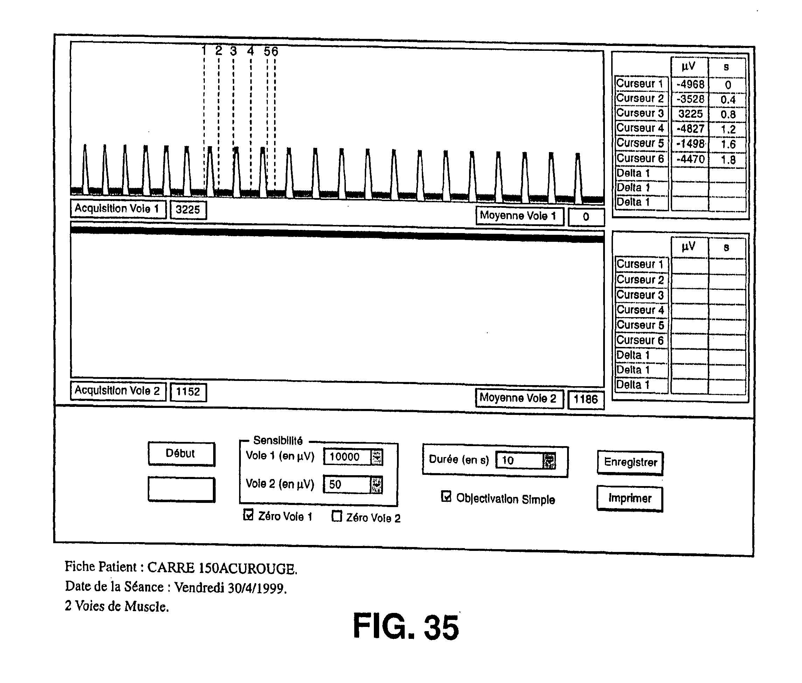

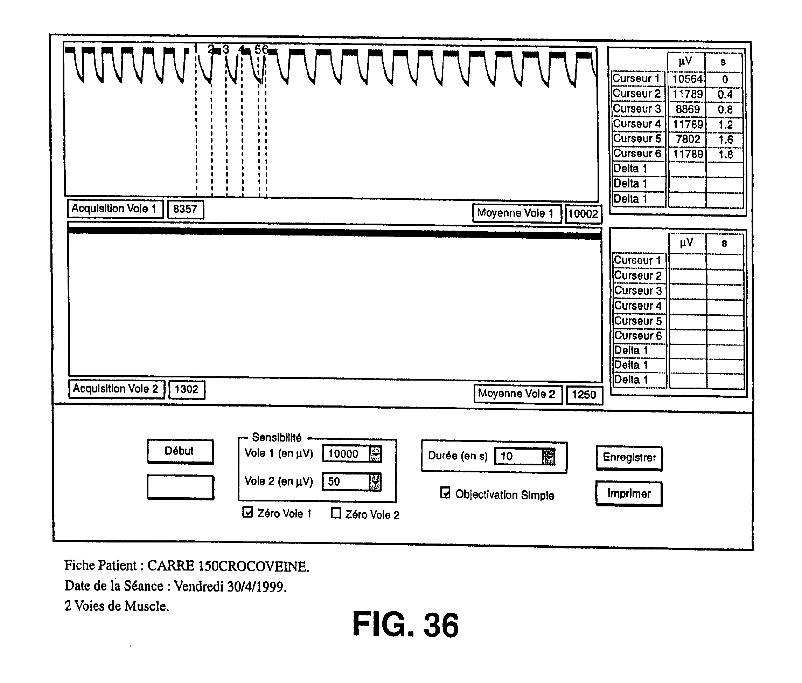

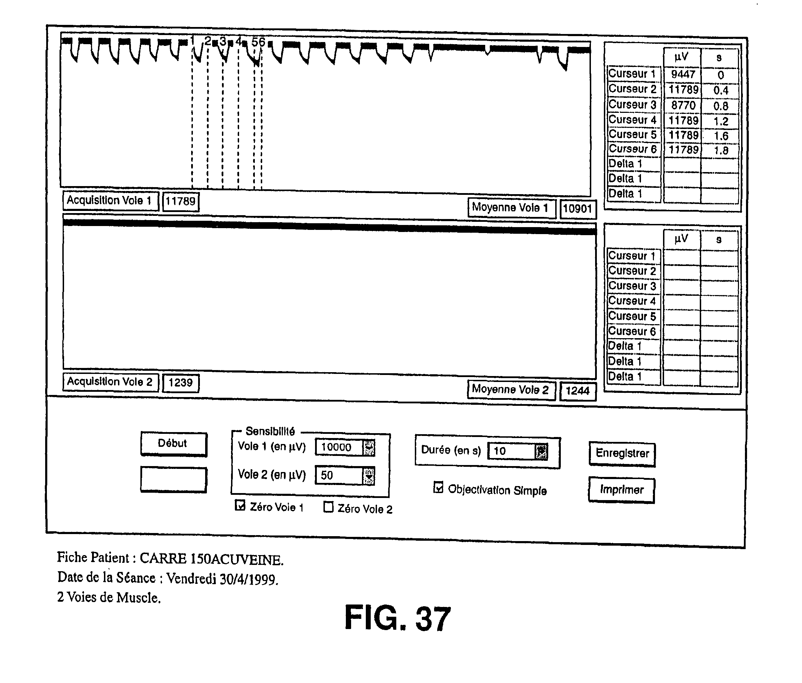

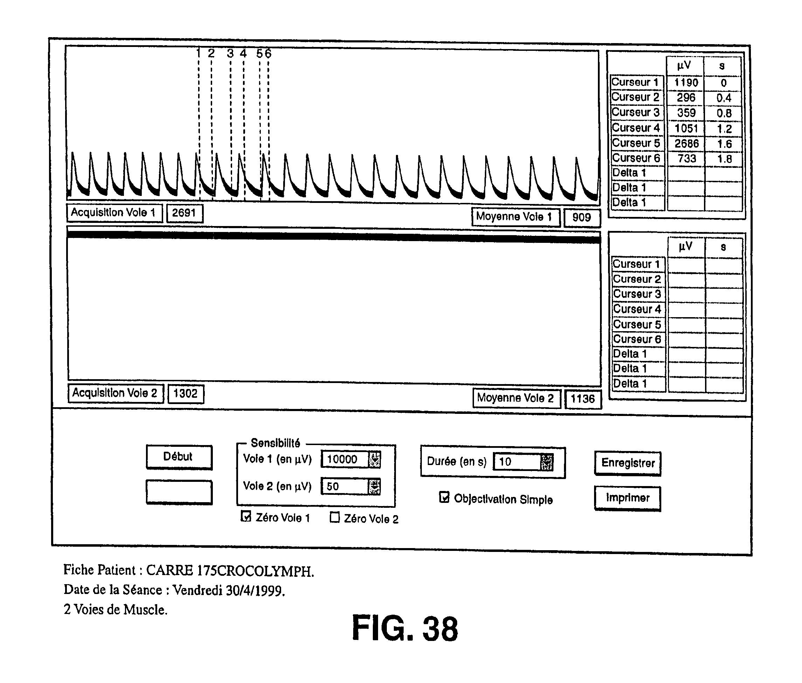

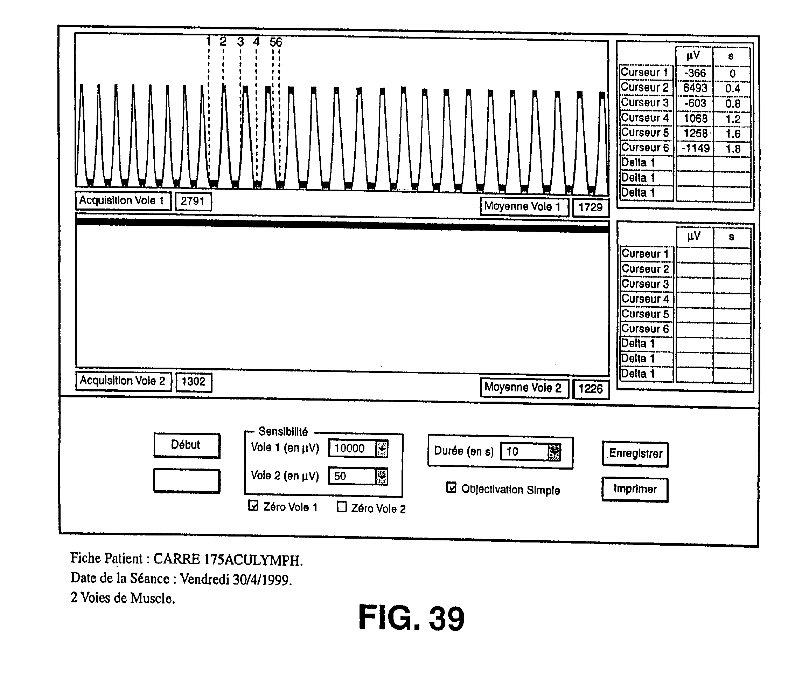

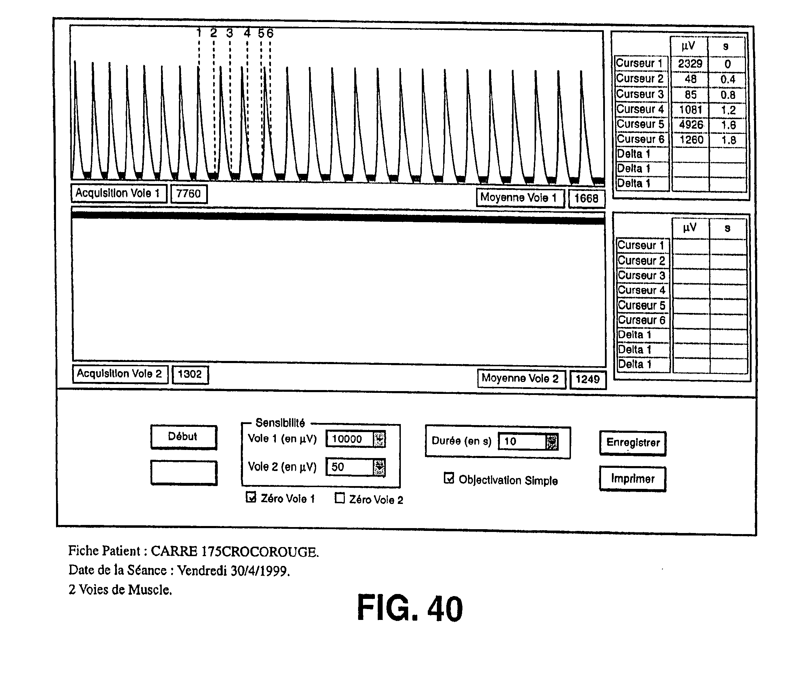

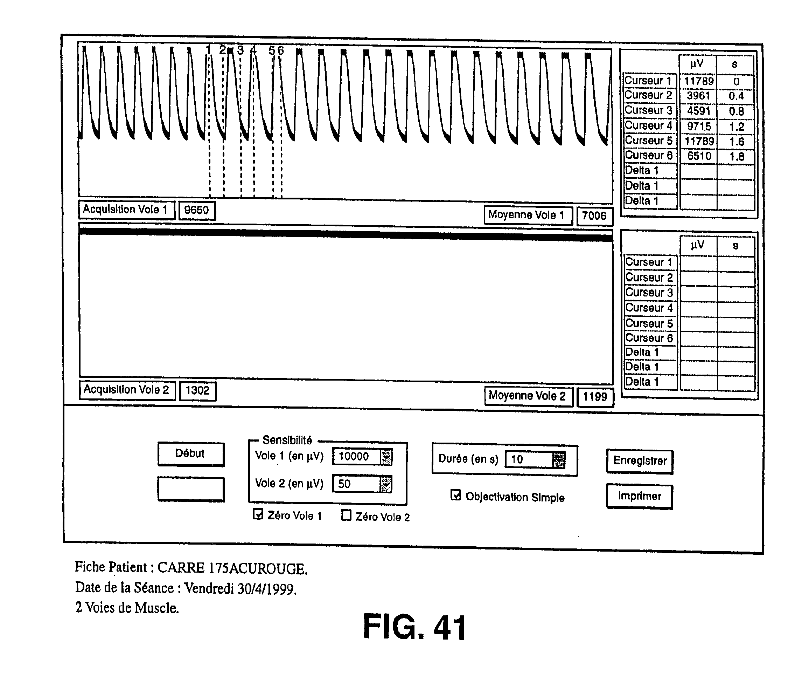

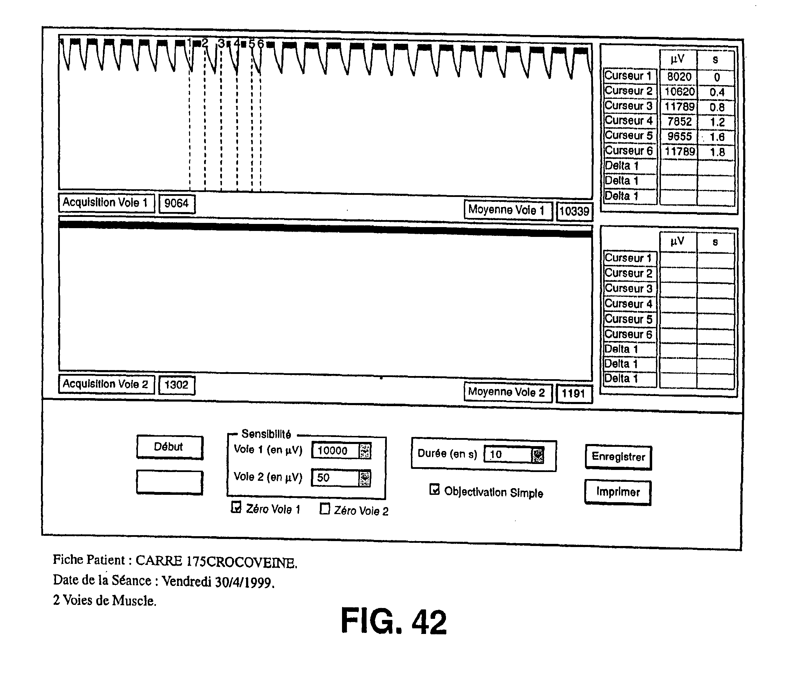

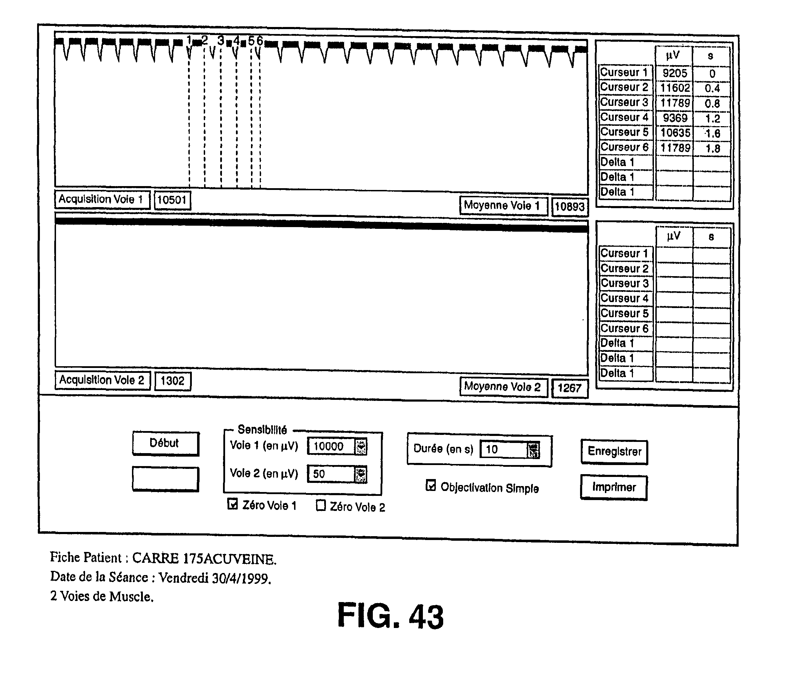

- les figures 1 à 43 représentent les tracés d'activité EMG obtenus avec l'appareil selon l'invention et avec des appareils de l'art antérieur, comme spécifié dans les ESSAIS ci-dessous.
- la figure 44 représente un « lymphangion ».
- la figure 45, qui se compose des figures 45 A et 45 B, représente la structure des tissus autour du système lymphatique.

### ESSAIS

### Mode opératoire général:

On a prélevé trois pièces anatomiques sur des porcs fraîchement abattus (5 minutes), soit : le coeur, certains vaisseaux sanguins, poumons, intestin grêle.

On effectue la dissection et le prélèvement des pièces anatomiques suivantes:
- veine cave 10 cm / 40 g
- canal thoracique (canal lymphatique majeur) et intestin grêle pour un total de 150 g
- muscles rouges squelettiques 150 g

Des essais ont également été effectués sur des êtres humains sains (référence « SUJET » dans ce qui suit).

Les essais ont été menés sur un appareil de type EMG avec « biofeedback » (contrôle visuel graphique de la contraction) de type « YSY EST »^{TM} de la société YSY MEDICAL^{TM} capable d'effectuer des mesures d'EMG (activité - électromyogramme) et comportant un système de filtres destiné à n'enregistrer que l'activité des tissus (notamment contractions) et non les signaux parasites.

On a appliqué les impulsions, selon les cas, soit par des aiguilles plantées dans le tissu d'essai (essai référencé dans ce qui suit : « ACU »), soit par des pinces « crocodile » pinçant les tissus d'essai (« CROCO »).

Cette procédure d'essai a été jugée apte à simuler correctement les procédures de stimulation « externe non intrusive », comme notamment par des électrodes de contact, qui seront mises en oeuvre en pratique, sauf cas particuliers précisément identifiés.

Les essais ont visé à comparer les effets de divers appareils à différents réglages, afin de déterminer les formes et caractéristiques de courant capables de traiter ou stimuler le système lymphatique selon les objectifs précités.

L'activité a été calculée par une formule de « moyenne » entre les impulsions positives et négatives, établie en tenant compte de, et par rapport à, l' »acquisition de voie » qui apparaît en fenêtre à gauche des tracés, comme le reconnaîtra l'homme de métier.

### 5 ESSAI N° 1

Essai comparatif du Datavein^{™} et de l'appareil référencé FDLP selon l'invention.

L'appareil est réglé selon les instructions d'emploi de l'appareil Datavein^{™}, soit sur 1,75 Hz (fréquence de chaque impulsion), un temps de travail de 4 ms, une intensité de 6 microA et un temps de repos de 567 ms, avec 8/8 impulsions électriques.

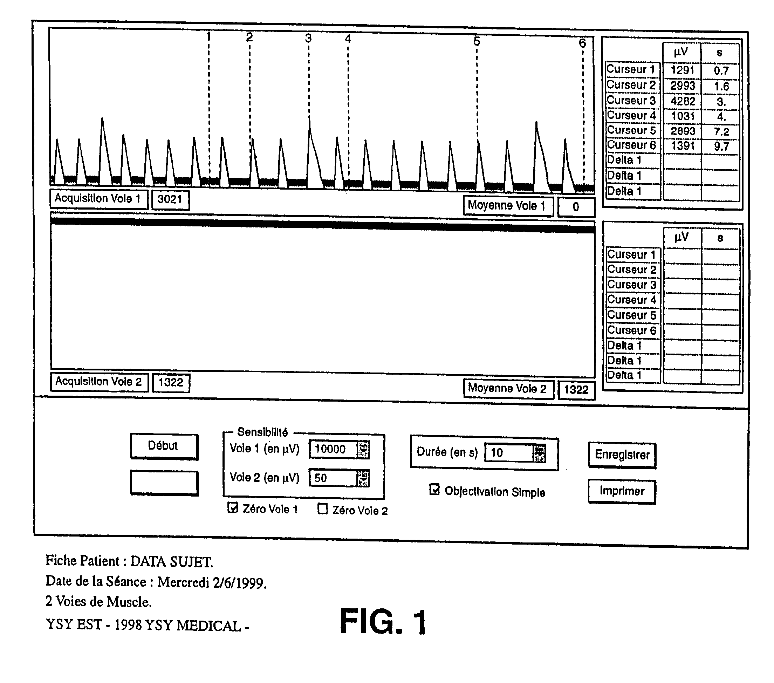

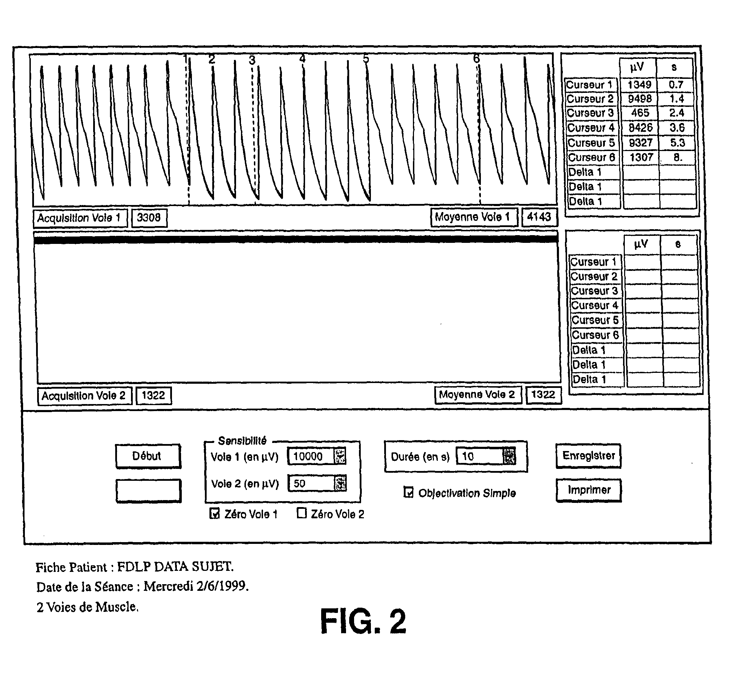

La figure 1 représente les résultats EMG obtenus avec l'appareil Datavein^{™} et la figure 2 les résultats obtenus avec l'appareil FDLP, aux mêmes réglages.

Le positionnement de curseurs permet de calculer des moyennes représentatives et reproductibles.

On voit que la moyenne d'activité EMG est de 2900 pour le Datavein^{™} et de 4143 pour le FDLP, soit une activité environ deux fois plus puissante pour le second appareil, aux mêmes réglages.

On note également la forme très différente des tracés.

### ESSAI N° 2

On a comparé les appareils Datavein^{™} et FDLP.

L'appareil FDLP a été réglé aux réglages préconisés pour l'appareil Datavein (TM) (ce qui est schématisé par la référence « FDLP DATA »).

L'essai a été effectué sur le tissu lymphatique ou de type lymphatique de porc (tissu lymphatique et intestin grêle) (référence « LYMPH ») et au moyen de pinces crocodile (référence « CROCO »).

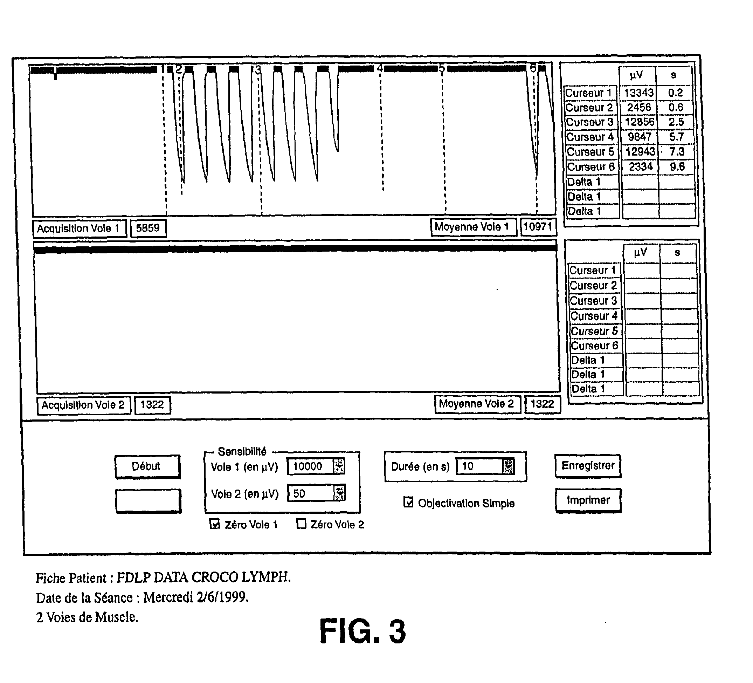

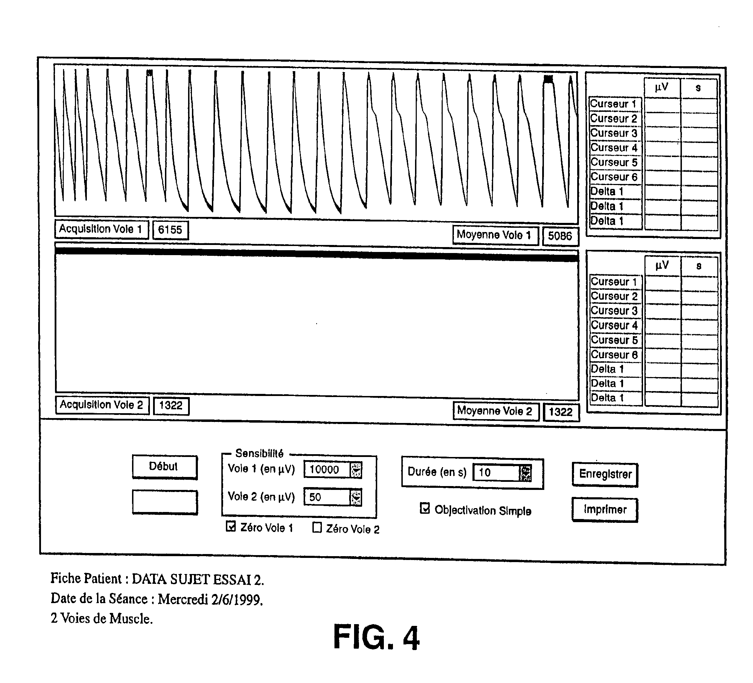

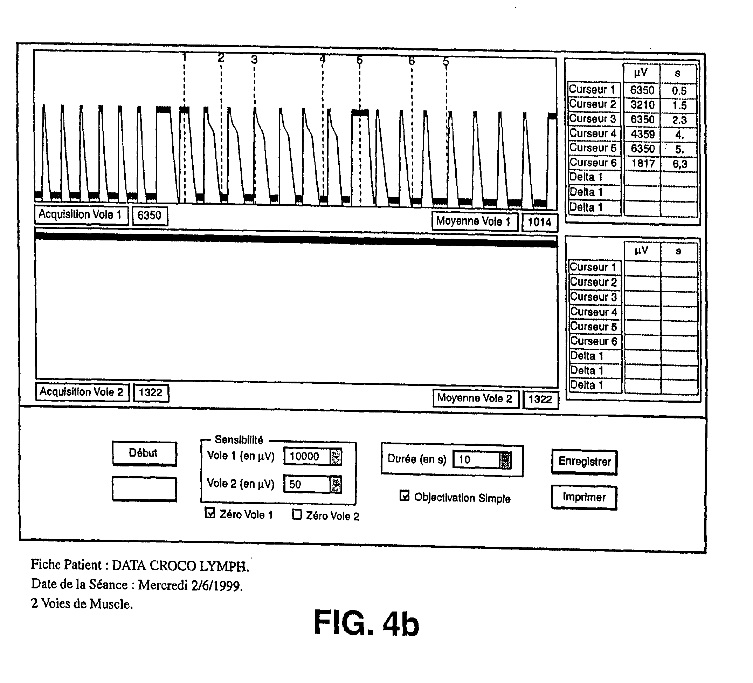

L'activité EMG avec le Datavein (TM) est de 1014 (figure 4b) et de 10971 (figure 3) avec le FDLP, lequel se révèle donc présenter un effet dix fois plus puissant.

On remarque encore la forme différente des impulsions et, avec le Datavein (TM), on observe des impulsions régulières, tandis qu'avec le FDLP on observe des impulsions alternées par groupes de 8 avec inversion de polarité, et pas avec le Datavein (TM).

### ESSAI N° 3

On a comparé les appareils Datavein (TM) et FDLP.

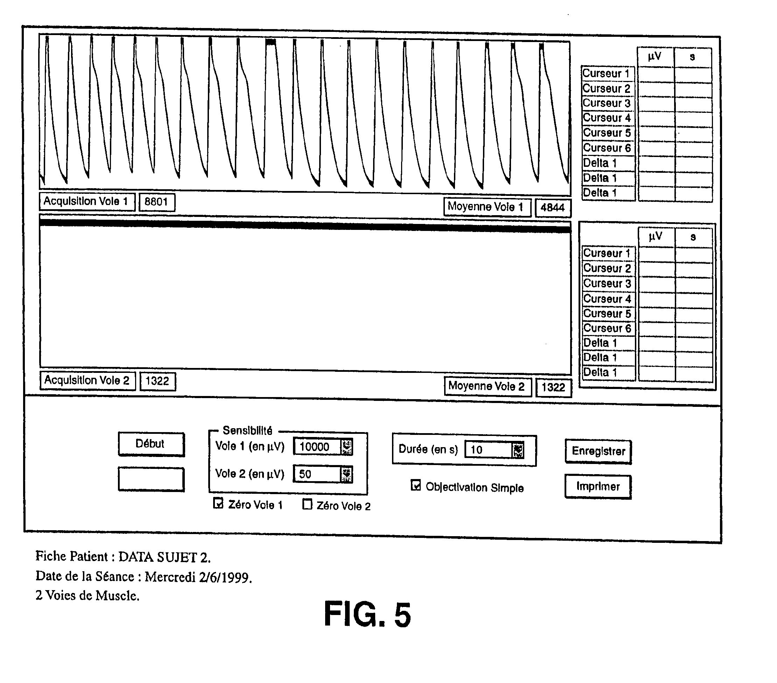

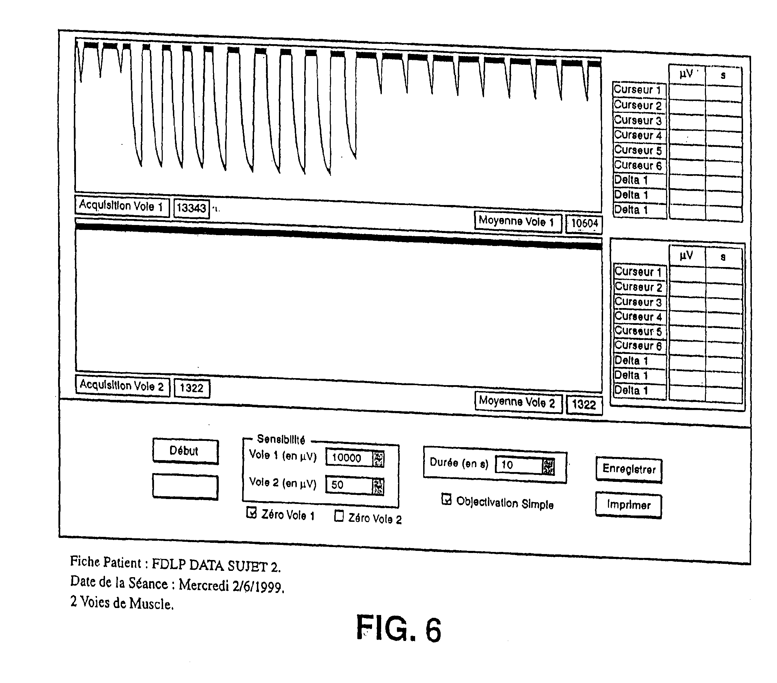

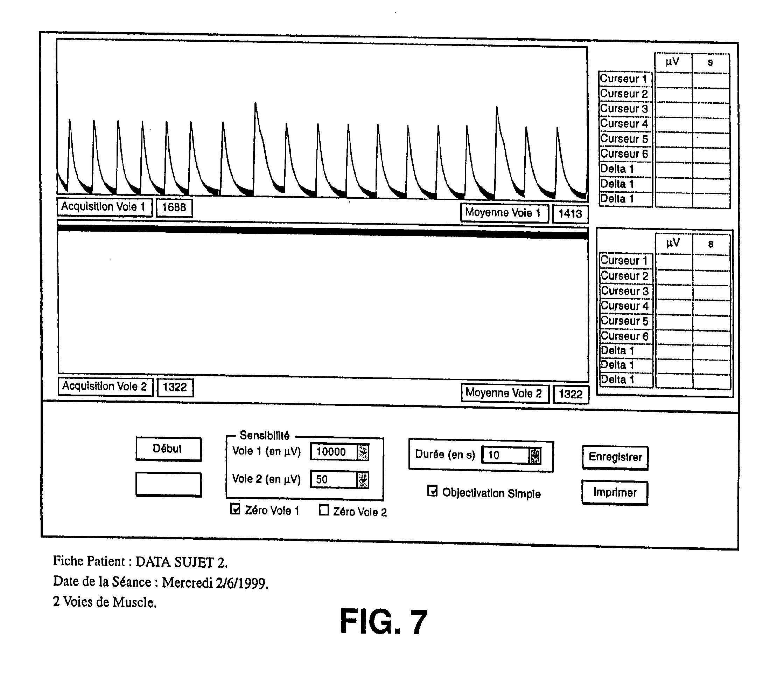

L'essai a été effectué sur un être humain (« SUJET ») par le Datavein (TM) avec deux essais (figures 4 et 5) et par le FDLP réglé aux réglages du Datavein (TM) (figure 6), ainsi encore que par le Datavein (TM) (figure 7).

Les appareils sont réglés à 30 microA.

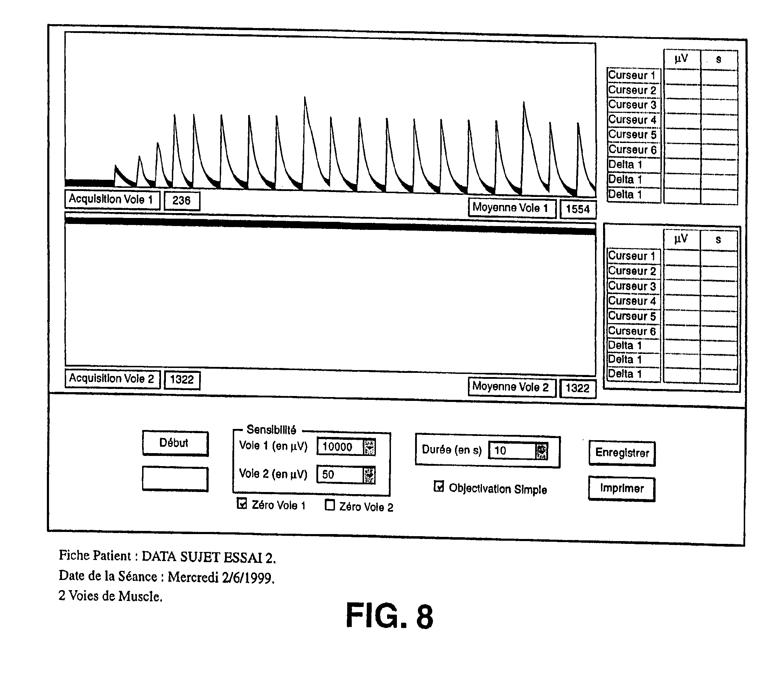

On note que l'activité avec le FDLP réglage Datavein (TM) est de 10604 (figure 6) alors qu'avec le Datavein (TM) il est de 1413 (figure 7), 1554 (figure 8), 5086 (figure 4), 4844 (figure 5).

Le FDLP est donc de 2 à 7 fois plus puissant que le Datavein (TM) dans cet essai.

### ESSAI N° 4

On a cherché dans cet essai à comparer le « ressenti » physique d'un sujet humain, lorsque traité par le Datavein (TM) ou par le FDLP.

On s'est aperçu que, pour retrouver le ressenti du FDLP réglé à seulement 30 microA, il a été nécessaire de monter l'intensité délivrée par le Datavein (TM) jusqu'à 150 microA.

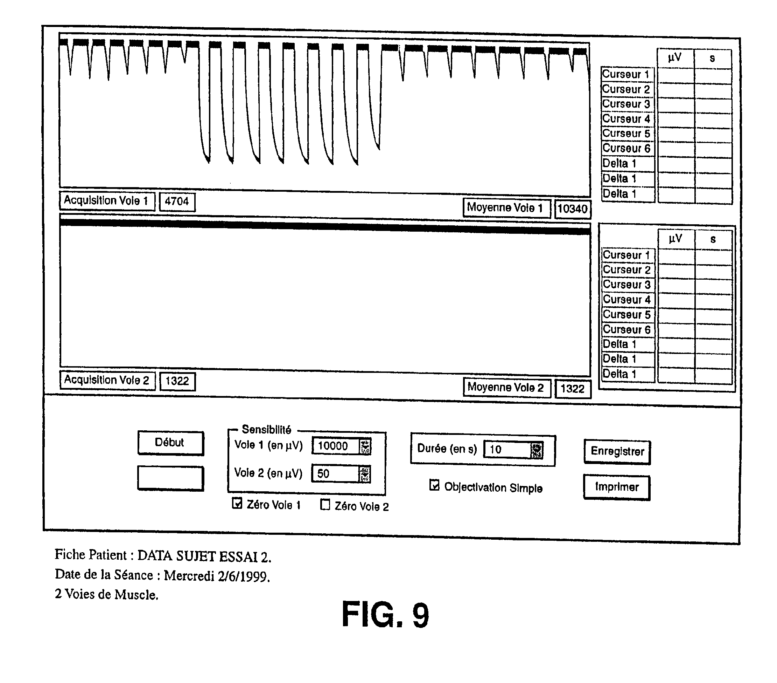

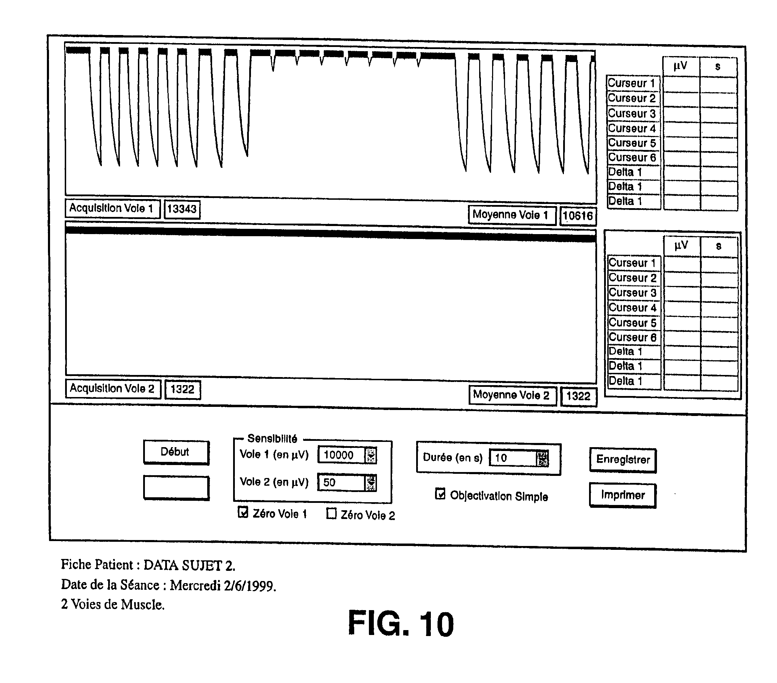

Ceci est confirmé par les figures 9 et 10 (Datavein (TM) réglé à 154 microA ; EMG moyenne 10340 ou 10616).

On effectuera une comparaison utile avec la figure 6 (FDLP réglé aux paramètres préconisés pour le Datavein (TM)) qui donnait une activité moyenne de 10604 à 30 microA alors que, à la même intensité, le Datavein (TM) n'en produisait que 1413.

### ESSAI N° 5

La supériorité globale du FDLP ayant été démontrée ci-dessus dans diverses configurations, on a commencé avec cet essai à rechercher les meilleurs caractéristiques de courant pour le FDLP.

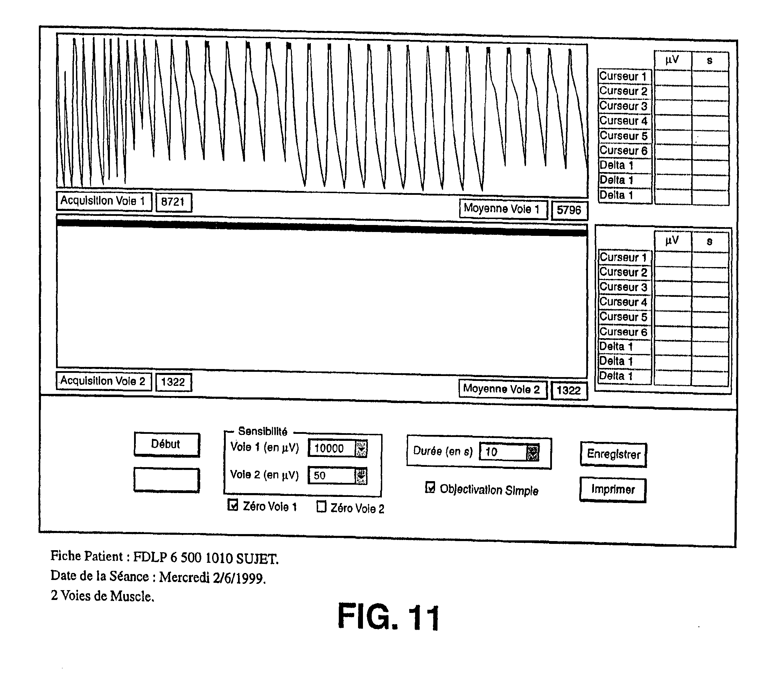

La figure 11 représente le tracé d'activité sur un sujet du FDLP réglé à 6 ms de temps de travail, 500 ms de temps de repos, trains ou « salves » 10/10 et intensité 6 microA.

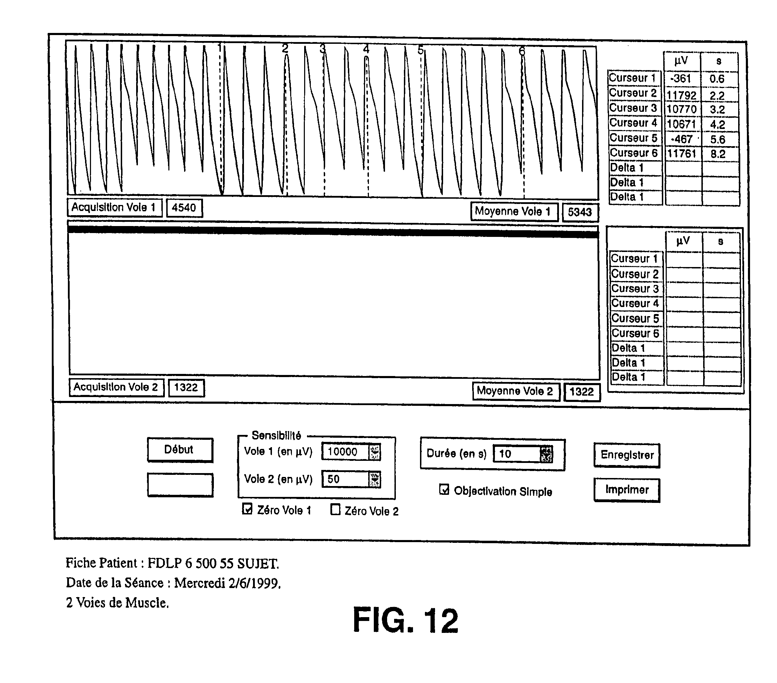

La figure 12 représente le même tracé, sauf en ce que les trains sont de 5/5 au lieu de 10/10.

Pour le tracé 10/10, on observe une activité moyenne de 5796, contre 5343 pour le tracé 5/5.

Les deux activités sont donc sensiblement équivalentes, mais le réglage à 10/10 produit chez le sujet un « ressenti » plus confortable qu'à 5/5.

On a cherché à expliquer ce phénomène, et, sans vouloir être lié par une quelconque théorie, le demandeur considère que le réglage 10/10 est mieux adapté au rythme lent des battements naturels du système lymphatique. Pour stimuler manuellement un ganglion, le praticien effectue de 6 à 8 battements par minute, ce qui correspond à une fréquence de l'ordre de 0,1 Hz. Un collecteur lymphatique effectue de 10 à 12 mouvements par minute, soit une fréquence de l'ordre de 0,2 Hz.

Le réglage du FDLP à 10/10, avec 6 ms de travail et 650 ms de repos, à 6 microA, donne une fréquence de l'ordre de 1,52 Hz.

Le réglage du FDLP à 10/10, avec 2 ms de travail, 500 ms de repos, à 6 microA, donne une fréquence de l'ordre de 1,99 Hz.

Il semble que la combinaison de ces deux fréquences et des trains d'impulsions soient en harmonie avec le rythme lent naturel du système lymphatique.

Le réglage préconisé pour le Datavein (TM) est de 1,75 Hz.

Datavein (TM) ans le même réglage, le Datavein (TM) ne donne que des valeurs de 2900 ou 1413 (figures 1 à 6 microA et 7 à 30 microA), et révèle donc dans cet essai une efficacité encore inférieure de moitié à celle du FDLP.

### ESSAI N° 6

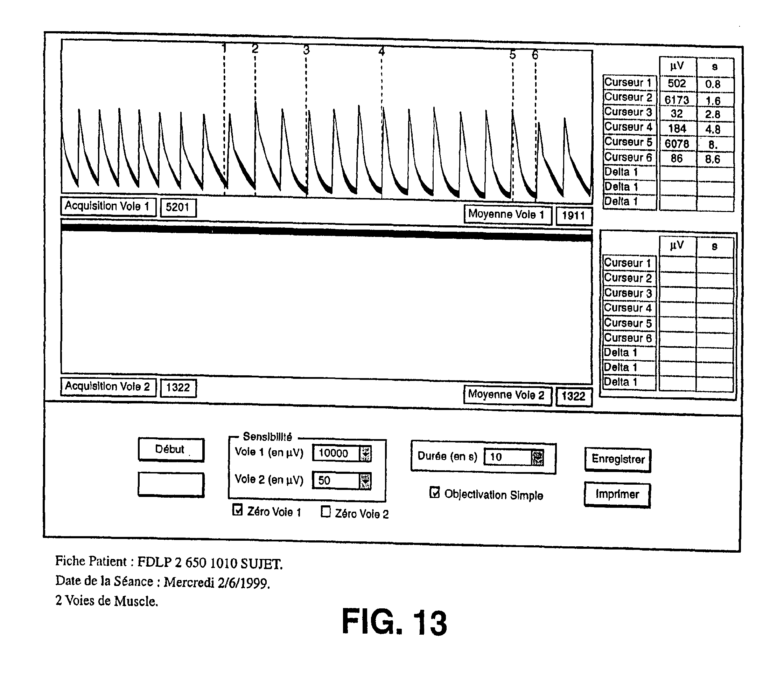

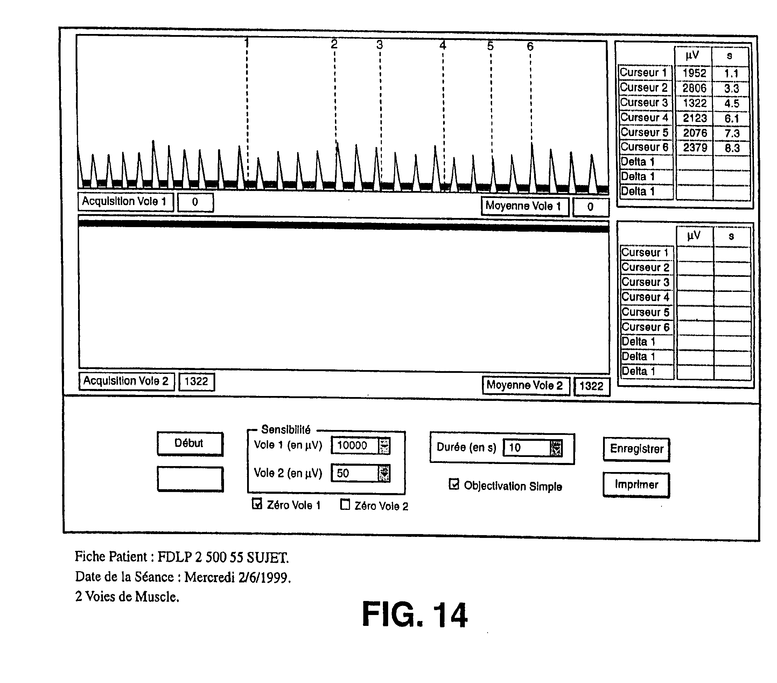

On a encore comparé dans cet essai deux réglages différents de FDLP, l'un (figure 13) sur un sujet humain à 2 ms de travail, 650 ms de repos et trains à 10/10, à 6 microA, et l'autre (figure 14) sur un sujet humain à 2 ms de travail, 500 ms de repos et trains à 5/5.

L'efficacité moyenne obtenue sur la figure 14 est de 677, contre 1911 sur la figure 13.

Il semble donc plus efficace de prévoir un temps de repos plus long pour le même temps de travail.

Il semble également plus intéressant de prévoir des trains à 10/10, ce qui est une confirmation d'un essai précédent.

### ESSAI N°7

On a effectué un essai du FDLP sur un sujet humain avec un réglage à 6 ms de temps de travail, 650 de temps de repos, et trains à 10/10, à 30 microA.

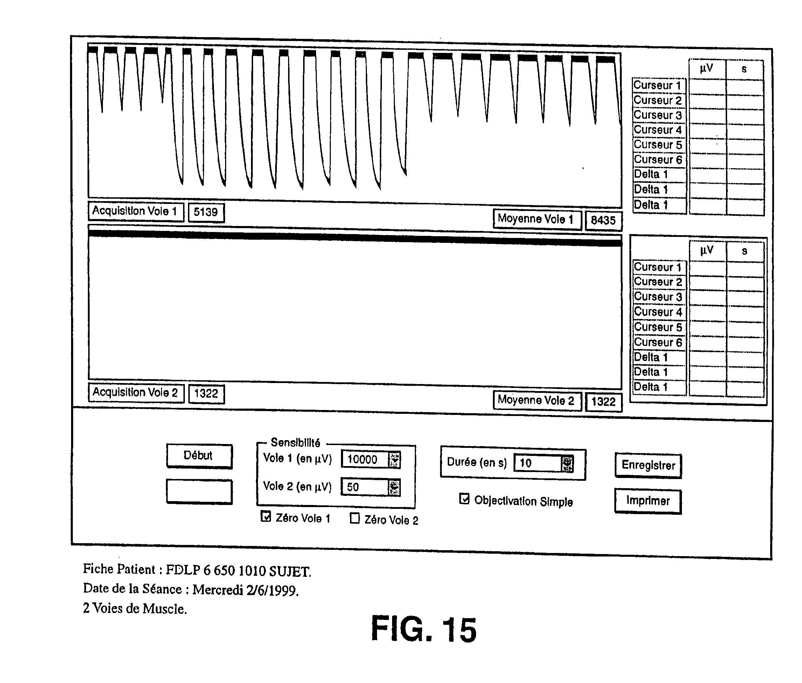

On obtient une activité EMG de 8435 (figure 15).

### CONCLUSION INTERMEDIAIRE

Les essais ci-dessus permettent de dégager une efficacité de 2 à 10 fois plus grande pour le FDLP que pour le Datavein (TM), d'une part, et d'autre part révèlent pour le FDLP la possibilité de régler l'appareil de façon à générer deux types de courants :
- un premier type de courant est un courant « régulateur ou rééducateur», utile principalement pour le traitement des pathologies du système lymphatique, et qui sera centré sur un réglage d'environ :
   2 ms de travail 500 ms de repos trains 10/10 >> 1,99 Hz
- un second type de courant « stimulateur » ou « turbo », provoquant une stimulation du système lymphatique beaucoup plus forte que le précédent, utile principalement dans les autres applications du FDLP, travail physiologique, et qui sera centré sur un réglage d'environ :
   6 ms de travail 650 ms de repos trains 10/10 >> 1,52 Hz.

### ESSAI N° 8

On a effectué des essais sur un tissu veineux, soit avec des aiguilles plantées, soit en pinçant des pinces crocodile.

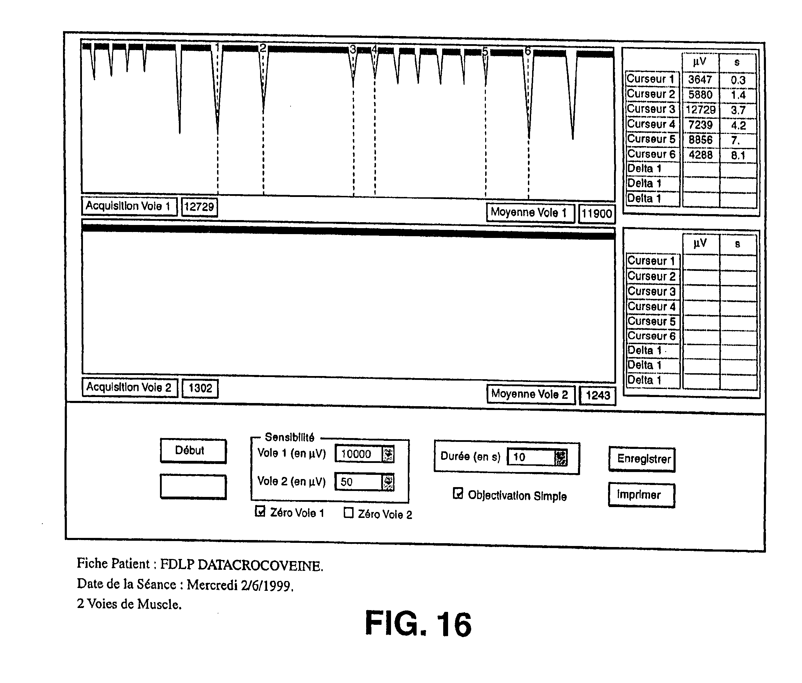

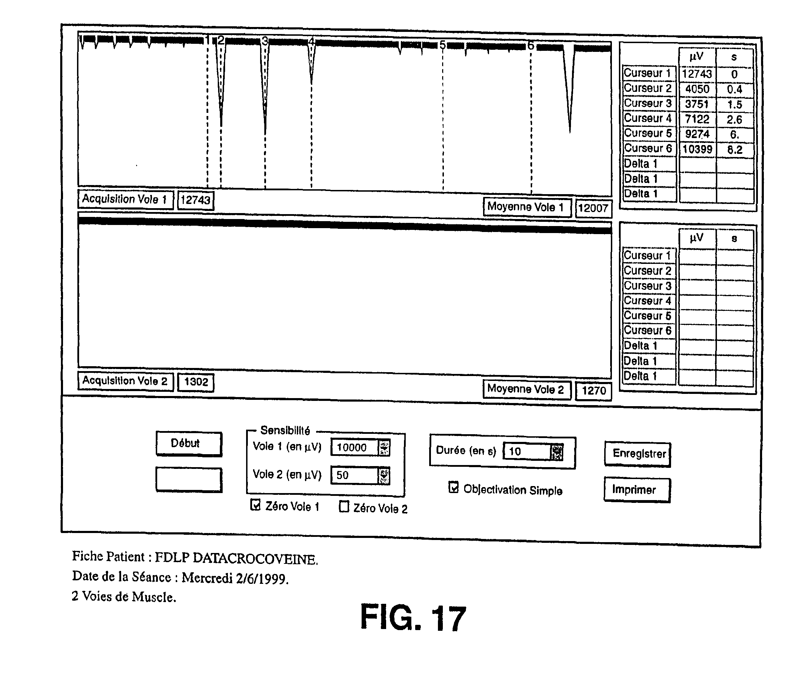

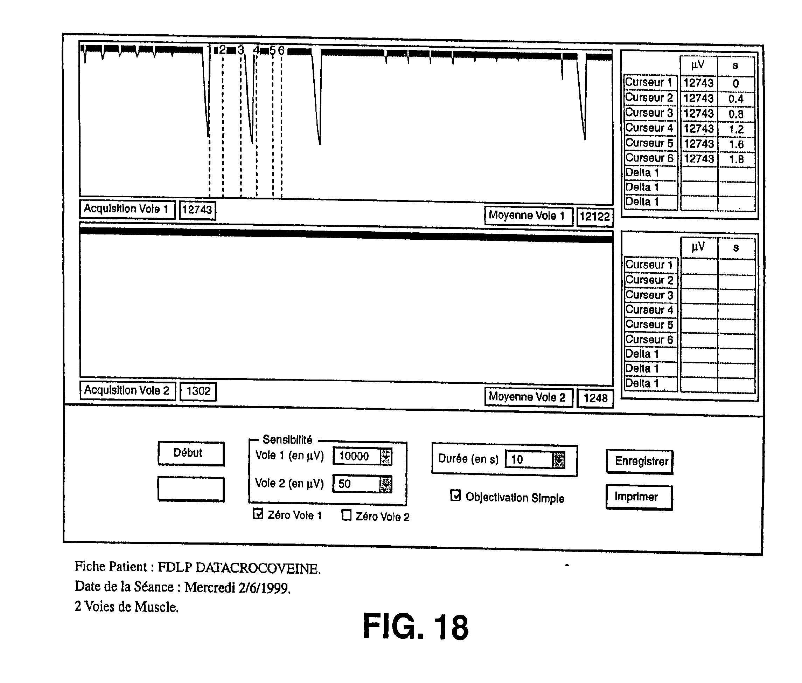

On a utilisé pour obtenir les traçés obtenus sur les figures 16, 17 et 18 un FDLP réglé Datavein (TM) (« FDLP DATA ») avec des pinces crocodile (« CROCO ») et sur un tissu veineux (« VEINE »). On a obtenu des activités de 11900, 12007 et 12122.

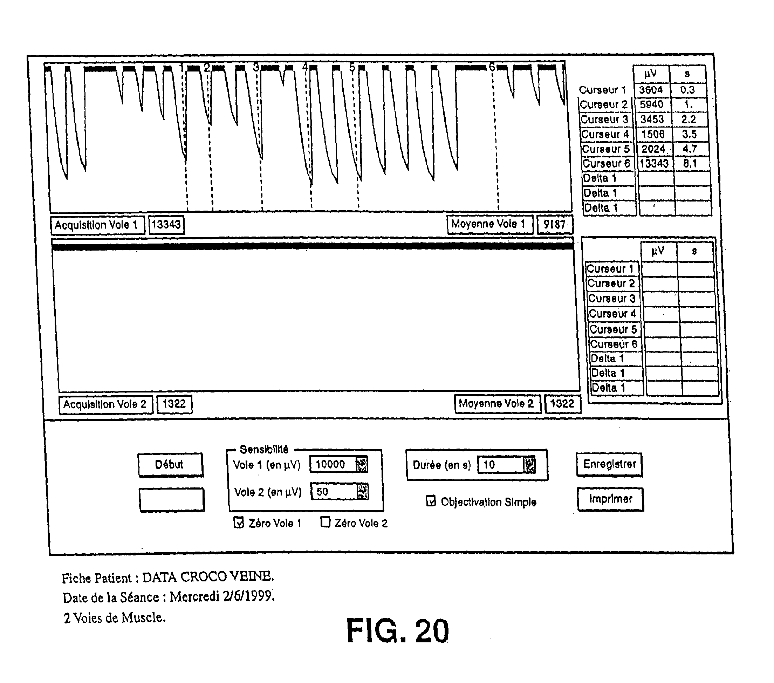

Afin d'effectuer une comparaison avec le Datavein (TM) , on a également effectué un essai avec le Datavein (TM), aux réglages préconisés par le constructeur, sur un tissu veineux et avec des pinces crocodile (figure 20). On a obtenu une valeur de seulement 9187.

Même sur un tissu veineux, le FDLP est donc nettement plus performant que le Datavein (TM).

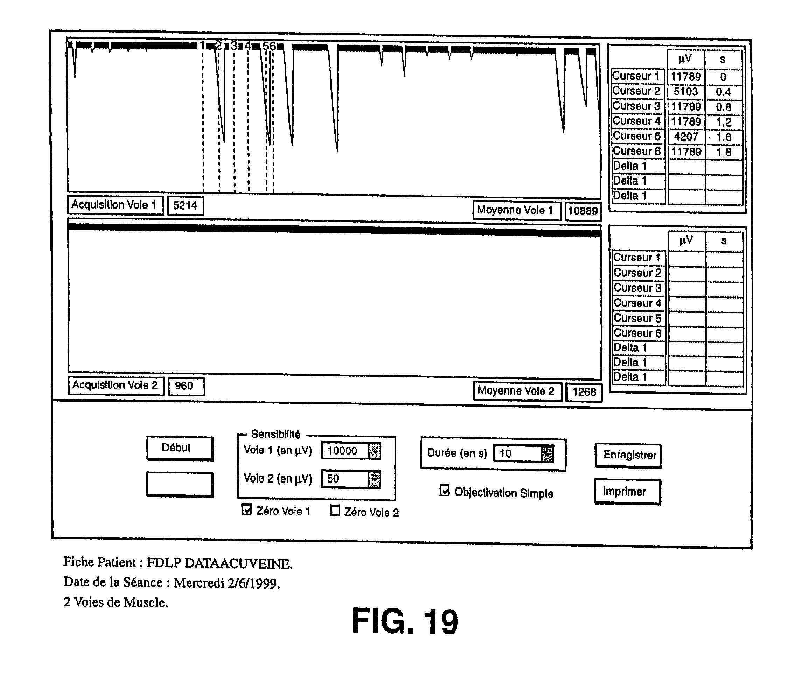

On a également effectué un essai du FDLP réglé Datavein (TM) sur du tissu veineux mais en plantant des aiguilles (« ACU ») (figure 19). On a obtenu une valeur de 10889, donc inférieure aux valeurs obtenues avec les pinces crocodile. Ceci semble infirmer la théorie du brevet Klotz selon laquelle les veines seraient composées de muscles blancs. En effet, dans un tel cas, l'effet ACU aurait dû être plus important que l'effet CROCO.

### ESSAI N° 9

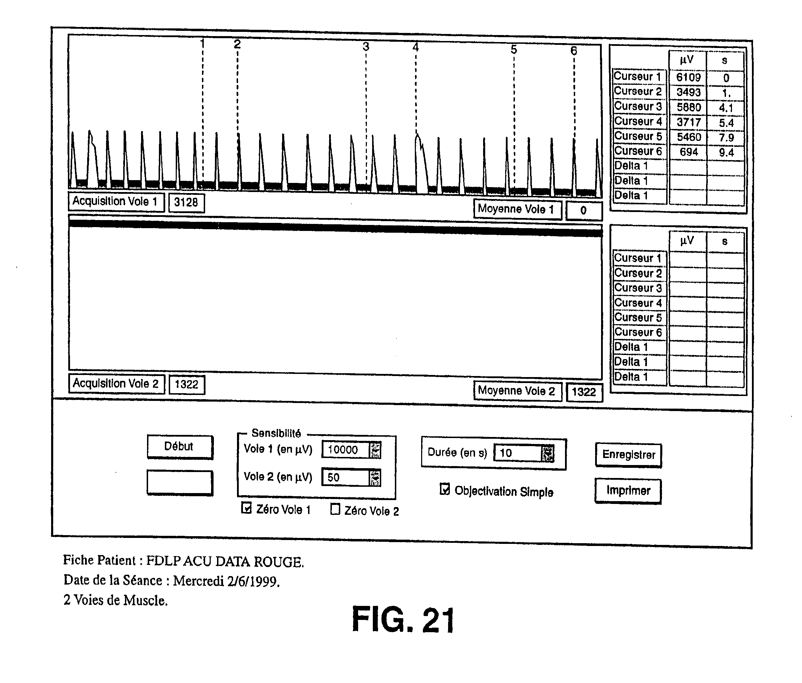

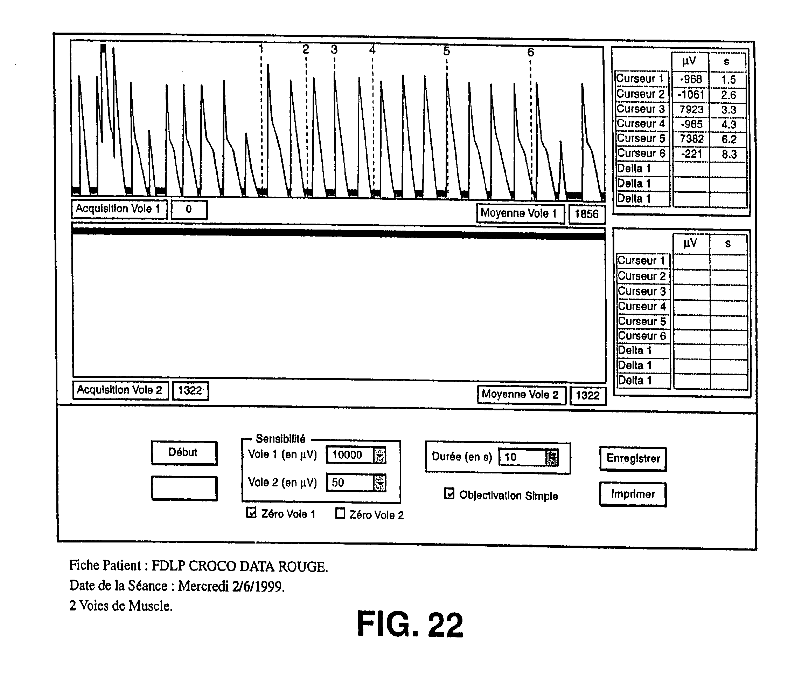

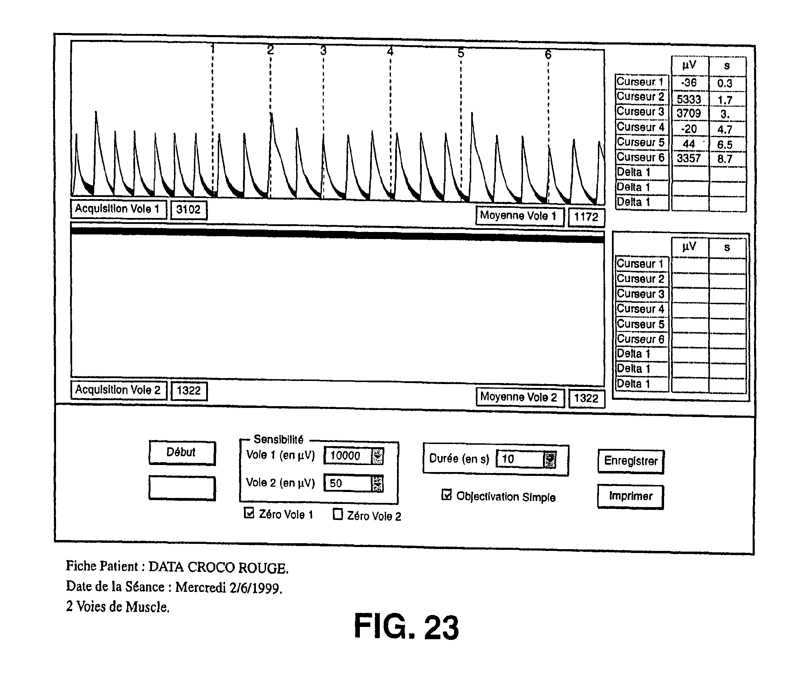

On a effectué des essais :
- sur muscle rouge avec FDLP réglé Datavein (TM), avec aiguilles (figure 21). Activité (moyenne) : 5344.
- sur muscle rouge avec FDLP réglé Datavein (TM), avec pinces crocodile (figure 22). Activité 1856
- sur muscle rouge avec Datavein (TM), avec pinces crocodile (figure 23). Activité 1172. Le résultat obtenu avec Datavein (TM) est donc inférieur à celui obtenu avec FDLP.

### ESSAI N° 10

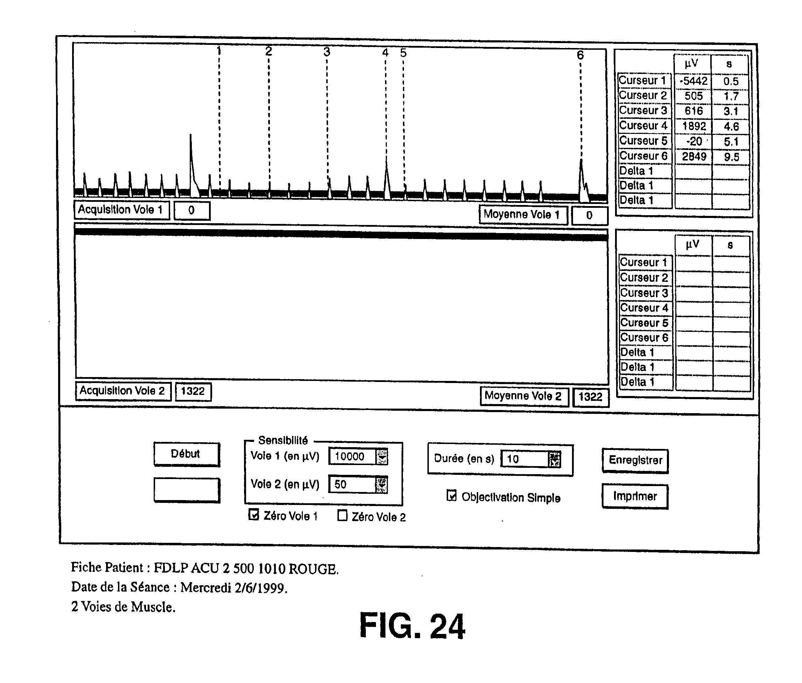

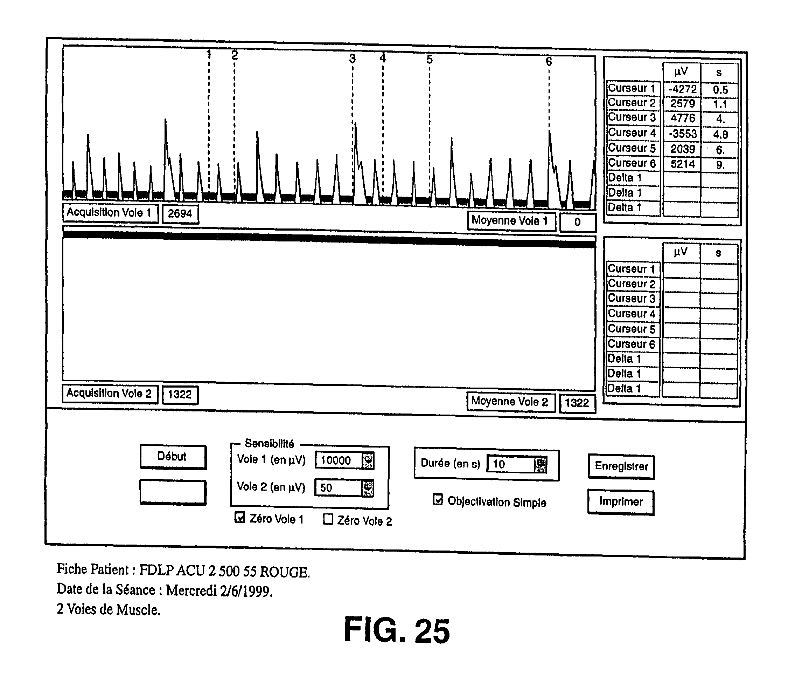

On a effectué des essais de courant dit « régulateur » avec le FDLP:
- sur muscle rouge, avec FDLP, réglé 2 ms de travail, 500 ms de repos, aiguilles et trains 10/10 (figure 24): activité: 2730
- sur muscle rouge, avec FDLP, réglé 2 ms de travail, 500 ms de repos, aiguilles et trains 5 / 5 (figure 25): activité: 3215

On voit nettement sur les tracés que le réglage 5/5 est supérieur au réglage 10/10 pour le muscle rouge, alors que la constatation est inverse pour le tissu lymphatique (réglage 10/10 plus performant pour le tissu lymphatique).

### ESSAI N° 11

On a effectué des essais de signaux carrés.

On a utilisé l'appareil MICROSTIM^{™} de la société Physio-INSEP^{™}, dont le constructeur déclare qu'il fonctionne en « veino-pompe » ou par effet de pompe sur le système strié rouge squelettique, périphérique au système veineux (par des contractions - relaxations) à un premier réglage de 1,25 Hz.

On a obtenu les tracés :

| | |
|---|---|
| Croco sur tissu lymphatique (figure 26) | résultat médiocre |
| Aiguilles sur tissu lymph. (figure 27) | résultat un peu meilleur |
| Croco sur muscle rouge (figure 28) | bon résultat 3554 |
| Aiguilles sur muscle rouge (figure 29) | bon résultat 2323 |
| Croco sur tissu veineux (figure 30) | 9567 |
| Aiguilles sur tissu veineux (figure 31) | 1584 |

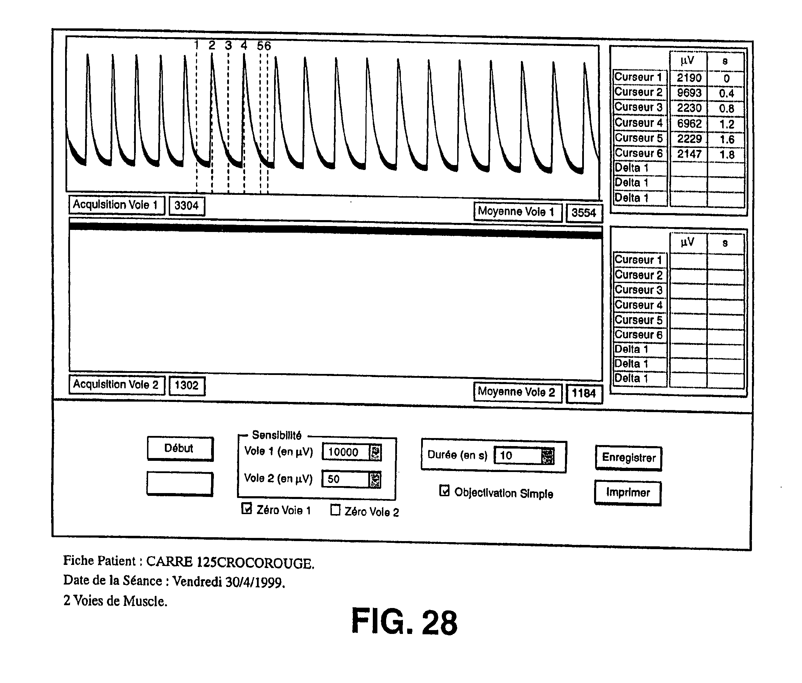

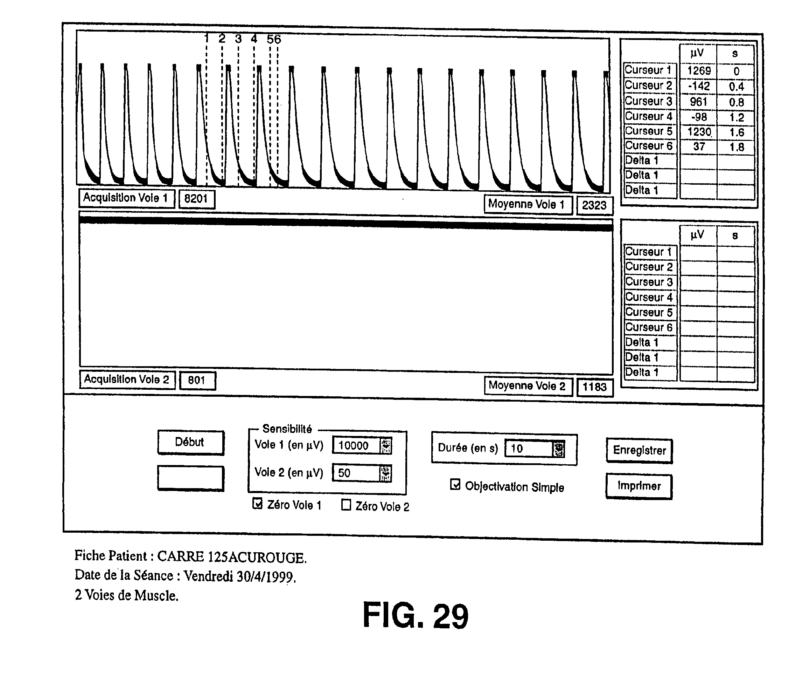

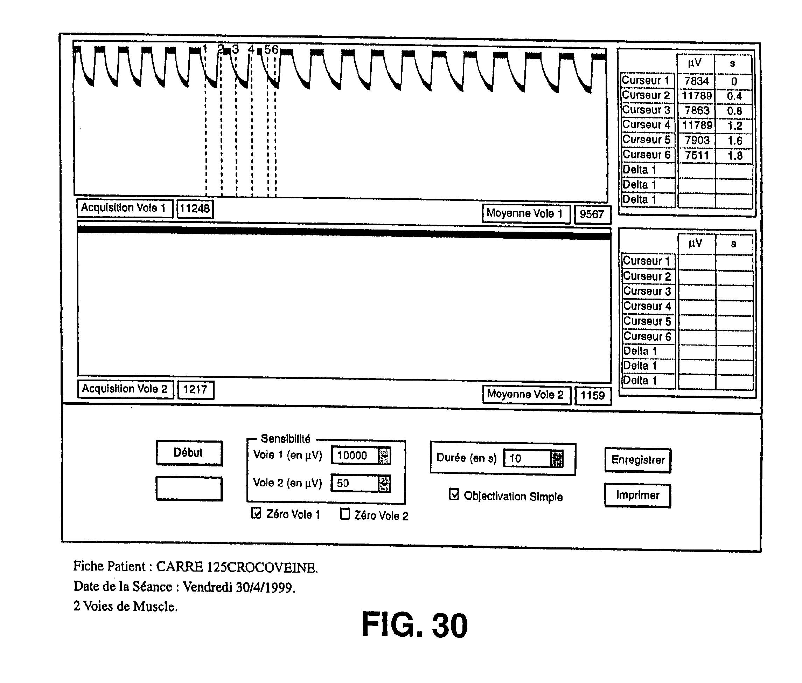

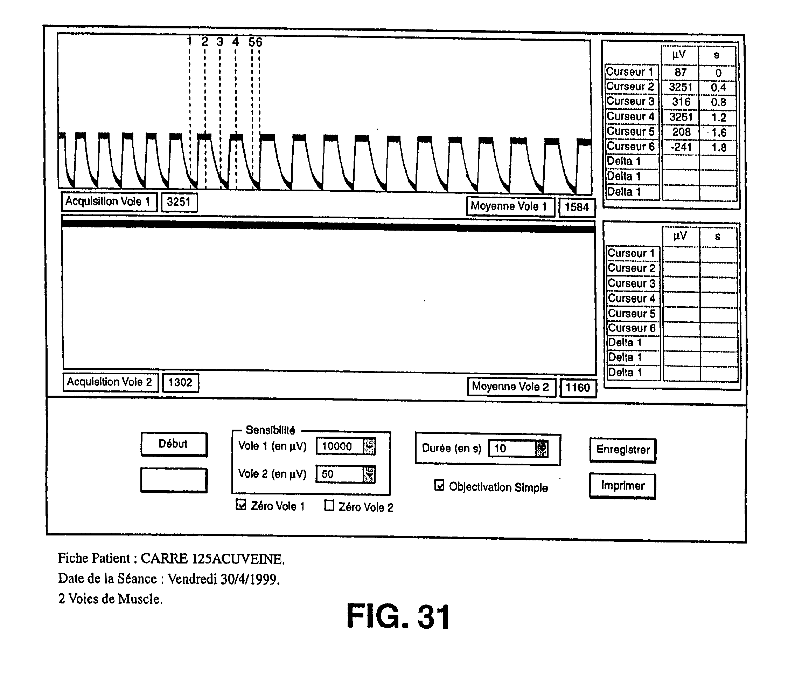

Les bons résultats des figures 28 à 31 étaient prévisibles, car l'appareil est destiné à un travail de « veino-pompe » en stimulant les muscles striés squelettiques périvasculaires.

On a ensuite utilisé l'appareil MICROSTIM^{™} à un second réglage de 1,50 Hz.

On a obtenu les tracés :

| | |
|---|---|
| Croco sur tissu lymphatique (figure 32) | résultat médiocre 1345 |
| Aiguilles sur tissu lymph.(figure 33) | 7551 problème d'aiguille |
| Croco sur muscle rouge (figure 34) | bon résultat 3779 |
| Aiguilles sur muscle rouge (figure 35) | bon résultat mais inférieur |
| Croco sur tissu veineux (figure 36) | 10 002 très bon résultat |
| Aiguilles sur tissu veineux (figure 37) | 10 901 très bon résultat |

On en déduit que l'application des signaux carrés est mieux adaptée au traitement du système veineux, car la stimulation touche les muscles striés squelettiques périvasculaires et non pas ou peu la médiaveineuse qui est quasiment absente sur l'ensemble des sytèmes veineux.

On a ensuite utilisé l'appareil MICROSTIM^{™} à un troisième réglage de 1,75 Hz qui est le réglage préconisé pour le Datavein (TM) par son constructeur.

On a obtenu les tracés : 1

| | |
|---|---|
| Croco sur tissu lymphatique (figure 38) | résultat très médiocre 909 |
| Aiguilles sur tissu lymph. (figure 39) | résultat moyen 1729 |
| Croco sur muscle rouge (figure 40) | résultat moyen 1668 |
| Aiguilles sur muscle rouge (figure 41) | bon résultat 7006 |
| Croco sur tissu veineux (figure 42) | 10 339 très forte activité |
| Aiguilles sur tissu veineux (figure 43) | 10 893 très forte activité |

Bien que la faible quantité de muscles rouges périvasculaires ait pu déformer certains résultats dans cet essai, ces essais multiples montrent que l'appareil agit très nettement sur la veine et un muscle rouge (à la façon d'une veino-pompe, avec un effet fort de contraction) que sur le système lymphatique où l'effet est quasi nul.

Ceci indique que les signaux carrés n'ont pas d'effet significatif sur le système lymphatique.

Ces essais ont également le mérite de valider l'ensemble des autres essais effectués plus haut, car ils permettent de retrouver des résultats annoncés par le constructeur. On peut donc en déduire que les essais effectués sur le FDLP et sur le Datavein (TM) sont significatifs.

L'invention concerne donc un appareil pour la stimulation et le traitement du système lymphatique (ce vocable recouvrant par simplicité tous les effets et toutes les applications mentionnés plus haut), du type comportant un générateur d'impulsions électrique et au moins deux électrodes que l'on place sur l'épiderme du sujet à traiter, caractérisé en ce que les impulsions de courant électriques ne sont pas formées de signaux carrés.

Selon un mode de réalisation particulier, l'appareil est caractérisé en ce que les impulsions de courant électrique présentent un temps de travail compris entre 1 et 12, de préférence 1 et 8 ms, de préférence 2 ou 6 ms.

Selon un mode de réalisation particulier, l'appareil est caractérisé en ce que les impulsions de courant électrique présentent un temps de repos compris entre 300 et 900 ms, de préférence 400 et 700 ms, de préférence 500 ou 650 ms.

Selon un mode de réalisation particulier, l'appareil est caractérisé en ce que les impulsions de courant électrique présentent une intensité de courant de 6 à 300 microA, de préférence 6 à 50 ou 100 ou 150 microA, , ou plus, en fonction de la sensibilité (« ressenti ») du patient et du nombre d'électrodes (2, 4, 6, etc.... cf. ci-dessous), comme le comprendra aisément l'homme de métier.

Selon encore un mode de réalisation particulier, l'appareil est caractérisé en ce que les impulsions de courant électrique présentent une fréquence d'environ 0,1 à 3 Hz, de préférence d'environ 1,99 Hz ou d'environ 1,52 Hz.

Selon encore un mode de réalisation particulier, l'appareil est caractérisé en ce que les impulsions de courant électrique présentent des trains d'impulsions.

Selon encore un mode de réalisation particulier, l'appareil est caractérisé en ce que les impulsions de courant électrique présentent de préférence des trains d'impulsions de type 10/10.

Selon encore un mode de réalisation particulier, l'appareil est caractérisé en ce que les impulsions de courant électrique présentent des trains d'impulsions avec inversion de polarité entre chaque train.

L'appareil est caractérisé en ce que les impulsions de courant électrique sont réglées à :

| | |
|---|---|
| Temps de travail | 2 ms |
| Temps de repos | 500 ms |
| Trains d'impulsion | 10/10 |
| Fréquence | 1,99 Hz |

ce qui produit un courant « régulateur » ou de « rééducation » pour le traitement des systèmes lymphatiques pathologiques ou déficients.

L'appareil est caractérisé en ce que les impulsions de courant électrique sont réglées à :

| | |
|---|---|
| Temps de travail | 6 ms |
| Temps de repos | 650 ms |
| Trains d'impulsion | 10/10 |
| Fréquence | 1,52 Hz |

ce qui produit un courant « stimulateur » ou de « turbo », donnant un effet très fort de stimulation du système lymphatique. Ce courant sera donc plus apte à accélérer ou activer le système lymphatique non pathologique.

Selon encore un mode de réalisation particulier, l'appareil est caractérisé en ce que les impulsions de courant électrique sont appliquées par :
- des électrodes cutanées « de contact », de type plat, carbonées ou non, collantes ou non, avec ou sans hydrogel, posées ou collées sur l'épiderme
et éventuellement, dans certains cas identifiés précisément par le praticien
- des pinces de type « crocodile »
- des aiguilles plantées dans le tissu dermique

Selon encore un mode de réalisation particulier, l'appareil est caractérisé en ce que les impulsions de courant électrique sont appliquées par au moins deux électrodes.

Selon encore un mode de réalisation particulier, l'appareil est caractérisé en ce que les impulsions de courant électrique sont appliquées par x couples d'électrodes (+)(-), par exemple deux électrodes (+)(-) ou quatre électrodes (+)(-) et (+)(-) couvrant deux zones de traitement, etc.....

Les applications de l'invention, appareil et procédé, seront à titre non limitatif :
- la stimulation et le drainage lymphatique chez l'homme et l'animal, notamment, chez ce dernier, le cheval et autres animaux, comme notamment le chien, et certaines applications spécifiquement humaines comme :
   en esthétique :
      - le traitement de la cellulite
      - les soins du visage, (oedème, peau dénutrie)
      - cicatrices
   en pathologie :
      - la lymphostase telle que décrite ci-dessus ;
      - les troubles veineux
      - surcharges / récupération musculaire
      - cicatrices
      - inflammations, tendinites
      - lymphologie / phlébologie / traumatologie / traumatologie du sport / rhumatologie / dermatologie / chirurgie / gériatrie / gastro-entérologie / gynécologie / obstétrique / traitement des brûlés.
   en immunologie ;
      et, chez le cheval ou autres animaux comme notamment le chien :
      - toutes les pathologies circulatoires, fourbures et autres pathologies de surcharge (récupération musculaire, cicatrices, inflammation, tendinites, tendinopathies dont les tendinites, pathologies musculaires ou ostéo-articulaires)
      - pathologie de bloc opératoire, chez le cheval
      et autres applications analogues humaines ou vétérinaires qui seront évidentes et accessibles à l'homme de métier.

## Revendications

1. Appareil de stimulation du système lymphatique chez l'être humain ou les animaux, notamment le cheval, y compris un générateur d'impulsions électriques et au moins deux électrodes que l'on place sur l'épiderme du sujet à traiter, de manière à faire passer dans le corps humain ou animal considéré des trains de stimulations électriques, chaque stimulation ou impulsion étant
**caractérisée en ce que** :
- il ne s'agit pas de signaux carrés ;
- le temps d'excitation est plus court que le temps de repos ou relaxation ;
- le temps d'excitation est compris entre 2 et 8 ms ;
- le temps de repos ou relaxation est compris entre 400 et 850 ms ;
étant adapté pour délivrer :
- un premier type de courant qui est un courant « régulateur ou rééducateur », utile principalement pour le traitement des pathologies du système lymphatique, et qui sera centré sur un réglage d'environ :
2 ms de travail 500 ms de repos trains 10/10 fréquence 1,99 Hz ;
et
- un second type de courant « stimulateur » ou « turbo », provoquant une stimulation du système lymphatique beaucoup plus forte que le précédent, utile principalement dans les autres applications de l'appareil, travail physiologique, et qui sera centré sur un réglage d'environ :
6 ms de travail 650 ms de repos trains 10/10 fréquence 1,52 Hz.

2. Appareil selon la revendication 1, **caractérisé en ce que** l'intensité du courant électrique appliqué sera inférieure ou égale à 1 mA, et sera de préférence de l'ordre de 6 à 300 µA de préférence de 6 à 50 ou 100 ou 150 en fonction du nombre d'électrodes.

3. Appareil selon l'une quelconque des revendications 1 ou 2, **caractérisé en ce que** chaque train d'impulsions présente une inversion de polarité par rapport au précédent.

4. Appareil selon l'une quelconque des revendications 1 à 3, **caractérisé en ce qu'**il comporte au moins deux électrodes cutanées « de contact » de type plat, carbonées ou non, collantes ou non, avec ou sans hydrogel, posées ou collées sur l'épiderme
ou
- des pinces de type « crocodile »
- des aiguilles adaptées à être plantées dans le tissu dermique.

5. Appareil selon l'une quelconque des revendications 1 à 4, **caractérisé en ce qu'**il comporte plusieurs couples d'électrodes, par exemple deux électrodes ou quatre électrodes couvrant deux zones de traitement.

## Claims

1. A device for stimulation of the lymphatic system in the human or animal, the horse in particular, **characterized in that** such device comprises a generator of electric pulses and at least two electrodes which are positioned on the epidermis of the subject to be treated so as to cause electric stimulation sequences to pass into the human or animal body involved, each stimulation or pulse being **characterized in that**:
- square wave signals are not involved;
- the excitation period is shorter than the rest or relaxation period;
- the excitation period ranges from 2 to 8 ms;
- the rest or relaxation period ranges from 400 to 850 ms;
being suitable for delivery of:
- a first type of current which is a "regulating" or "reeducating" current, useful primarily for treatment of pathological conditions of the lymphatic system and being about a setting of around:
2 ms of work, 500 ms of rest, 10/10 sequences, frequency 1.99 Hz
and
- a second type of "stimulating" or "turbo" current which causes stimulation of the lymphatic system much stronger than the preceding one, useful primarily in other applications of the device, physiological labor, and being about a setting of around:
6 ms of work, 650 ms of rest, 10/10 sequences, frequency 1.52 Hz.

2. The device as specified in claim 1, wherein the intensity of the electric current applied is lower than or equal to 1 mA, and is preferably of the order of 6 to 300 µA, preferably 6 to 50 or 100 or 150 or more, depending on the number of electrodes.

3. The device as specified in claim 1 or 2, wherein each sequence of pulses exhibits reversal of polarity relative to the preceding sequence.

4. The device as specified in one of claims 1 to 3, wherein such device comprises at least two "contact" electrodes of the flat type, carbonated or non-carbonated, adhesive or non-adhesive, with or without hydrogel, positioned on or adhering to the epidermis or
- "alligator" clips
- needles suitable for being implanted in the cutaneous tissue.

5. The device as specified in one of claims 1 to 4, wherein such device comprises several couples of electrodes, such as two electrodes or four electrodes covering two areas of treatment.

## Patentansprüche

1. Gerät zur Stimulation des Lymphsystems beim Menschen oder bei den Tieren, insbesondere beim Pferd, mit einem Generator von elektrischen Impulsen und zumindest zwei Elektroden, die derart auf der Epidermis des zu behandelnden Subjektes angebracht werden, dass dem entsprechenden menschlichen oder tierischen Körper Folgen von elektrischen Stimulationen zugeführt werden, wobei jede Stimulation oder jeder Impuls **dadurch gekennzeichnet ist, dass**:
- es sich nicht um Rechtecksignale handelt;
- die Erregungszeit kürzer als die Ruhe- oder Entspannungszeit ist;
- die Erregungszeit zwischen 2 und 8 ms einnimmt;
- die Ruhe- oder Entspannungszeit zwischen 400 und 850 ms einnimmt;
wobei es dazu geeignet ist, folgendes zu liefern:
- einen ersten Stromtyp, der ein "Regler- oder Rehabilitationsstrom" ist, der im Wesentlichen zur Behandlung der Pathologien des Lymphsystems nützlich ist, und der auf eine Einstellung zentriert ist, die ungefähr wie folgt ist:
2 ms Arbeitszeit 500 ms Ruhezeit Folgen 10/10 Frequenz 1,99 Hz;
und
- einen zweiten Stromtyp, einen "Stimulations- " oder "Turbostrom", der eine Stimulation des Lymphsystems bewirkt, die erheblich stärker, als die vorhergehende ist und die im Wesentlichen bei den anderen Anwendungen des Gerätes ,der physiologischen Arbeit, nützlich ist, und der auf eine Einstellung zentriert ist, die ungefähr wie folgt ist:
6 ms Arbeitszeit 650 ms Ruhezeit Folgen 10/10 Frequenz 1,52 Hz.

2. Gerät nach Anspruch 1, **dadurch gekennzeichnet, dass** die Intensität des angewandten elektrischen Stroms kleiner oder gleich 1 mA ist, und sich vorzugsweise auf 6 bis 300 µA beläuft, vorzugsweise von 6 bis 50 oder 100 oder 150 in Abhängigkeit von der Anzahl der Elektroden.

3. Gerät nach einem der Ansprüche 1 oder 2, **dadurch gekennzeichnet, dass** jede Impulsfolge eine Polaritätsumkehrung im Verhältnis zur vorhergehenden aufweist.

4. Gerät nach einem der Ansprüche 1 bis 3, **dadurch gekennzeichnet, dass** es zumindest zwei "Hautkontaktelektroden" des flachen Typs aufweist, die karbonisiert oder nicht karbonisiert, haftend oder nicht haftend, hydrogelhaltig oder hydrogelfrei, auf der Epidermis positioniert oder haftend sind,
oder
- Klammern des "Krokodiltyps",
- Nadeln, die dazu geeignet sind, in das Gewebe eingeführt zu werden.

5. Gerät nach einem der Ansprüche 1 bis 4, **dadurch gekennzeichnet, dass** es mehrere Paare von Elektroden aufweist, beispielsweise zwei Elektroden oder vier Elektroden, die zwei Behandlungszonen abdecken.
